# EUROPÄISCHE PATENTSCHRIFT

(11) **EP 2 957 283 B1**
(45) Veröffentlichungstag und Bekanntmachung des Hinweises auf die Patenterteilung: **21.12.2022**
(21) Anmeldenummer: 14173155.4
(22) Anmeldetag: 19.06.2014
(51) Int. Cl.: A61K 31/045, A61P 17/02, A61K 9/00, A61K 9/06, A61K 9/08, A61K 9/10, A61K 9/107, A61K 9/12

(54) **Verfahren zur Identifizierung von Medikamenten zur Beschleunigung der Wundheilung**
Process for identification of medicaments for acceleration of wound healing
Prodédé d'identification de médicaments pour l'accélération de la guérison de blessures

(43) Veröffentlichungstag der Anmeldung: 23.12.2015
(73) Patentinhaber: Symrise AG, 37603 Holzminden (DE)
(72) Erfinder: Hatt, Hanns, 44789 Bochum (DE); Conrad, Heike, 37085 Göttingen (DE); Busse, Daniela, 44329 Dortmund (DE)
(74) Vertreter: Fabry, Bernd

(56) Entgegenhaltungen:
- WO-A1-2008/068683
- WO-A1-2009/153572
- US-A- 5 759 556

## Beschreibung

### GEBIET DER ERFINDUNG

Die Erfindung befindet sich auf dem Gebiet der Pharmazie und betrifft Medikamente speziell zur Beschleunigung der Wundheilung, die spezielle Verbindungen mit einem Sandelholzgeruch enthalten.

### STAND DER TECHNIK

Mit Wundheilung bezeichnet man den körpereigenen Verschluss einer Wunde durch weitest gehende Wiederherstellung des beschädigten Körpergewebes. Es handelt sich dabei um einen natürlichen biologischen Prozess der bereits Minuten nach der Wundsetzung beginnt, wie mit enzym-histochemischen Verfahren nachgewiesen werden konnte. Die Blutplättchen treten an die geschädigte Stelle und versuchen sie zu verschließen. In manchen Fällen entsteht dabei über Exsudation (Flüssigkeitsabsonderung) eine Schorfbildung, was auch den oft mit der Wundheilung einhergehenden Juckreiz auslöst.

Die Wundheilung lässt sich in unterschiedliche Phasen einteilen. Nachdem das zerstörte Blutgefäß durch ein Gerinnsel verschlossen ist, finden zunächst keine weiteren makroskopisch oder mikroskopisch sichtbaren Reaktionen statt. Diese erste Latenzphase leitet über in Exsudationsphase, in der Fremdkörper und Keime durch den Austritt von Wundsekret aus der Wunde heraus geschwemmt werden. In dieser Phase sind die Zellen und Hormone des Immunsystems wesentlich beteiligt und zwar nicht nur zur Abtötung eingedrungener Bakterien oder Viren, sondern auch zur Stimulation des Heilungsprozesses selbst. Bei der Gerinnselbildung wird ein Fibrinnetz gebildet, welches ein Verkleben aneinander liegender Wundränder ermöglicht. Klares Wundsekret, welches aus Serum besteht, ist mit Entzündungszellen durchsetzt. Im Verlauf dieser Phase nimmt die Mitose im Wundgebiet zu. Monozyten reifen in dem Wundgebiet zu Makrophagen, die Zelltrümmer und Pfropf abräumen. Fibroblasten, die sich aus eingewanderten, aber auch aus im Wundrand ortständigen Bindegewebszellen entwickeln und durch Zellteilung vermehren, vollbringen in der folgenden Phase die eigentliche Aufbauarbeit.

In der folgenden Granulationsphase wird der Wunddefekt durch Proliferation von neuem Bindegewebe zunehmend aufgefüllt. Zeitgleich mit der zellreichen Auffüllung eines Wunddefektes erfolgt der Abbau des Fibrinnetzes (Fibrinolyse) durch Plasmin. Durch einsprossende Haargefäße erfolgt Vaskularisation, d.h. eine Zunahme der Anzahl an Gefäßen. Die Fibroblasten produzieren hexosaminhaltige saure Mucopolysaccharide, die als extrazelluläre Grundsubstanz des Bindegewebes fungieren und über intrazelluläre Vorstufen die schließlich extrazellulären kollagenen Bindegewebsfasern bilden. Der zeitliche Ablauf ist sehr komplex und unterliegt dem Einfluss zahlreicher Wachstumsfaktoren (Zytokine).

In der Regenerationsphase schließlich wird die Wunde an der Oberfläche durch Epithelisation geschlossen. Der Durchmesser einer gut granulierenden Wunde schließt sich zu einem Drittel ausschließlich durch Schrumpfung, zu zwei Dritteln durch Neubildung bzw. Zellteilung von Oberflächenzellen und Zellwanderung mit Hilfe des Fibrins vom Wundrand her zur Wundmitte. Das daruntergelegene Granulationsgewebe bildet zunehmend Kollagenfasern aus, womit die Wiederherstellung aller Hautschichten nahezu abgeschlossen ist. Die weitere Zunahme der Reißfestigkeit des Narbengewebes hängt von der Vernetzung, Verfestigung und Ausrichtung der Kollagenfasern ab. Der Wassergehalt des Gewebes nimmt ab, die anfänglich das Hautniveau gering überstehende Narbe schrumpft regelhaft unter Hautniveau. Auch nimmt der Gefäßreichtum des Narbengewebes ab. Die ursprünglich frisch rote Narbe wird weiß.

Aus dem Stand der Technik ist eine Vielzahl von pharmazeutischen Wirkstoffen bekannt, die die Wundheilung unterstützen. Beispielhaft genannt seien Aminothiazole **(**EP 0967980 B1**),** Arylsulfoaminopyrancarbonsäurehydroxymide **(**EP 1070058 B1**)** Cyanoanthranilamide **(**EP 1387838 B1**),** Antichymotrypsin Polypeptide **(**EP 1392354 B1**)** oder Dipyridyldihydropyrazole **(**EP 1877396 B1**).** Die Wirkmechanismen dieser Stoffe sind dabei völlig unterschiedlich: das Spektrum reicht von der Ausschüttung von Wachstumsfaktoren oder speziellen Interleukinen, über Inhibierung der HIF-Prolyl-4-hydroxylase bis hin zur Bereitstellung von Integrin Rezeptor Antagonisten.

Zu den natürlichen Stoffen, denen man unterstellt, dass sie die Wundheilung ebenfalls unterstützen, gehört seit langem das Sandelholzöl. Dieses ätherische Öl, das durch Wasserdampfdestillation aus dem Holz des tropischen Sandelbaums gewonnen wird, enthält eine Vielzahl von Spezies, deren Wirkung im Einzelnen bis heute nicht bekannt ist. Insbesondere ist unbekannt, welche Komponenten für die entzündungshemmenden Eigenschaften verantwortlich sind. Dabei ist zu berücksichtigen, dass natürliches Sandelholzöl aufgrund seines extensiven Einsatzes als Parfumrohstoff mittlerweile zu einem raren und sehr teuren Einsatzprodukt geworden ist.

Die Aufgabe der Erfindung hat folglich darin bestanden, die entzündungshemmenden Bestandteile des Sandelholzöls zu identifizieren, um auf diese Weise Medikamente zur Beschleunigung der Wundheilung zur Verfügung zu stellen, die insbesondere auch die Wundheilung unterstützen und vor allem die Zellproliferation sowie die Expression und Ausschüttung von Interleukin IL-1α stimulieren. Mit der Identifizierung der aktiven Spezies sollte gleichzeitig auch der Weg zur Synthese dieser Stoffe geebnet werden, um die natürlichen Ressourcen zu schonen und einen Beitrag zum Erhalt der Biodiversität zu leisten.

### BESCHREIBUNG DER ERFINDUNG

Die Erfindung ist in den angehängten Ansprüchen definiert. Ein erster Aspekt der Beschreibung betrifft ein Medikament enthaltend Derivate der 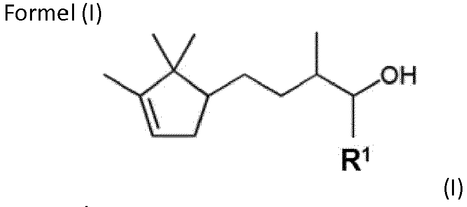 in der R¹ für Wasserstoff oder Methyl steht.

Ein zweiter Aspekt der Beschreibung betrifft ein Medikament enthaltend Derivate der Formel (I) 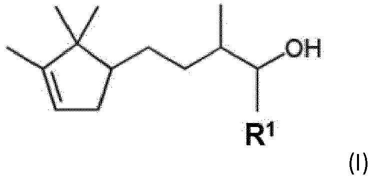 in der R¹ für Wasserstoff oder Methyl steht, zur Beschleunigung der Wundheilung.

Vorzugsweise handelt es sich bei den Derivaten der Formel (I) um Duftstoffe mit einem Sandelholzgeruch wie Sandalore^{®} (R¹= Methyl) oder Brahmanol^{®} (R¹ = Wasserstoff). Beide Stoffe sind im Markt frei erhältlich und werden bislang nur als Duftstoffe mit Sandelgeruch eingesetzt.

Überraschenderweise wurde gefunden, dass unter den bekannten Komponenten des Sandelholzöls sich keine unter den Testbedingungen als entzündungshemmend oder die Wundheilung beschleunigend erweist. Statt dessen wurden die beiden synthetischen Stoffe Sandalore und Brahmanol, die eine enge strukturelle Verwandtschaft zu den Bestandteilen des Sandelholzöls aufweisen und ebenfalls eine sandelholzartigen Geruch aufweisen, als die einzigen Agonisten für den unter anderem auch in der Haut befindlichen olfaktorischen Rezeptor OR2AT4 identifiziert, die diesen auch als einzige aktivieren können. In *Calcium Imaging*-Experimenten wurden 80 bis 90 % einer Probe von HaCaT-Zellen sowie der primären Keratinozyten repetitiv durch die beiden Stoffe stimuliert, wobei die Calciumsignale eine signifikante Sensitisierung, also eine Zunahme der Signalamplitude nach jeder weiteren Applikation zeigten.

Mit der Aktivierung des Rezeptors und der Auslösung der Signalkaskade ging eine Erhöhung der Proliferation und Migration der HaCaT-Zellen einher. Dabei zeigte sich, dass die beiden Stoffe die Phosphorylierung der MAP-Kinasen p38 und Erk1/2 stimuliert, die im Zusammenhang mit der epidermalen Wundheilung stehen. In einem *in vitro* Wund-Kratz-Assay konnte gezeigt werden, dass sich Kratzer bei HaCaT-Zellen um 26 % und bei Keratinozyten um 34 % schneller schließen, wenn die Zellen mit 500 µM Sandalore bzw. Brahmanol behandelt wurden.

Schließlich belegen die Ergebnisse, dass durch die beiden synthetischen Sandelholzduftstoffe auch die Expression sowie Ausschüttung von IL-1α erhöht sowie die Proteinkinase Akt, die an Differenzierungsprozessen von humanen Keratinozyten beteiligt ist, aktiviert wird.

Dieser Befund ist umso überraschender, als die beiden synthetischen Stoffe, die gerade keine Bestandteile des Sandelholzöls darstellen, an sich nur zur Referenzzwecken getestet werden sollten.

Aus den vorliegenden Ergebnissen kann nun aber sicher geschlossen werden, dass die Verabreichung von Sandalore^{®} und/oder Brahmanol^{®} vorzugsweise durch topische Anwendung auf der Haut zu einer Beschleunigung der Wundheilung führt, nämlich vor allem durch Aktivierung des Rezeptors OR2AT4 und die dadurch bewirkte Zellproliferation und Migration infolge einer Stimulierung der Phosphorylierung von MAP-Kinasen sowie durch eine erhöhte Interleukin IL-1α-Ausschüttung.

### MEDIKAMENTE

Die beschriebenen Medikamente die jedoch nicht Gegenstand der vorliegenden Erfindung sind, werden üblicherweise topisch appliziert. Dabei können sie beispielsweise als Lotionen, Cremes, Emulsionen, Gele, Salben sowie Sprays vorliegen. Ebenfalls ist es möglich entsprechende Verbandsstoffe, wie etwa Pflaster, mit diesen Zubereitungen zu tränken oder zu beschichten.

Die Einsatzmenge der Wirkstoffe bezogen auf die Endformulierung als Medikamente (also Wirkstoff plus pharmazeutisch zulässiger Träger und gegebenenfalls Zusatzstoffe) kann 0,001 bis etwa 2 Gew.-%, vorzugsweise etwa 0,01 bis etwa 1 Gew.-% und insbesondere etwa 0,1 bis etwa 0,5 Gew.-% betragen.

Die Medikamente können des Weiteren typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di- /Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:
**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
**Alkyl- und/oder Alkenyloligoglykoside.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
**Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
**Polyglycerinester.** Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.
3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

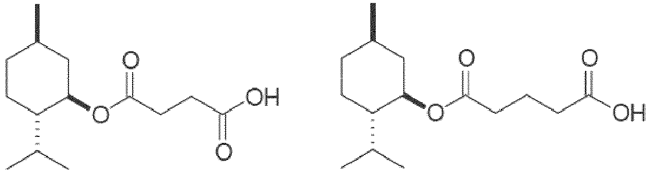

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (Lamequat^{®}L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine^{®}/Sandoz), Copolymere der Acrylsäure mit Dimethyl-diallylammoniumchlorid (Merquat^{®} 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäurean-hydrid-Copolymere und deren Ester, unvernetzte und mit Polyolen vernetzte Polyacrylsäuren, Acrylamidopropyltrimethylammoniumchlorid/ AcrylatCopolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/VinylacetatCopolymere, Vinylpyrrolidon/ Dimethylaminoethylmethacrylat/Vinylcaprolactam-Terpolymere sowie gegebenenfalls derivatisierte Celluloseether und Silicone in Frage.

### Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan⁸ AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol^{®} 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### SCREENINGVERFAHREN

Die Erfindung betrifft ein Verfahren zur Identifizierung von Wirkstoffen, die die Wundheilung begünstigen bzw. beschleunigen, bei dem man
(a) eine Keratinozytenkultur zu Verfügung stellt, die den olfaktorischen Rezeptor OR2AT4 enthält,
(b) der Kultur den zu testenden Wirkstoff hinzufügt und
(c) die Veränderung der intrazellulären Calciumkonzentration bestimmt.

Ein Wirkstoff weist dann eine Wahrscheinlichkeit auf, die Wundheilung zu unterstützen oder sogar zu beschleunigen, wenn der Rezeptor durch den Wirkstoff aktiviert wird und dadurch eine vermehrte Calciumausschüttung in die Zellen, vorzugsweise HaCaT-Zellen, stattfindet. Dies ist insbesondere dann der Fall, wenn mit jeder Gabe des Wirkstoffs die Amplitude der Calciumkonzentration im Vergleich zur vorherigen Verabreichung ansteigt.

### BEISPIELE

### CHARAKTERISIERUNG DES OR2AT4 IM HETEROLOGEN EXPRESSIONSSYSTEM

Um die Funktion eines Rezeptors in einem Gewebe aufklären zu können, ist es wichtig die Liganden zu kennen, um die Aktivierbarkeit des Rezeptors im nativen System zu gewährleisten. Die meisten OR sind bislang noch nicht deorphanisiert. Daher wurde der in humanen Keratinozyten identifizierte OR2AT4 zunächst in einem heterologen Expressionssystem genauer charakterisiert und das rezeptive Feld bestimmt.

### Heterologe Expression des OR2AT4 in Hana3A-Zellen

Als heterologes Expressionssystem wurden Hana3A-Zellen verwendet, die stabil endogene olfaktorische Transduktionsfaktoren exprimieren, welche gezielt den Einbau von OR in die Zellmembran unterstützen und die Aktvierbarkeit verbessern. Weiterhin wurden die 20 ersten N-terminalen Aminosäuren des Rhodopsins (Rho-*tag*) vor dem N-Terminus der codierenden Sequenz des ORs angefügt, wodurch die heterologe Expression ebenfalls unterstützt werden soll.

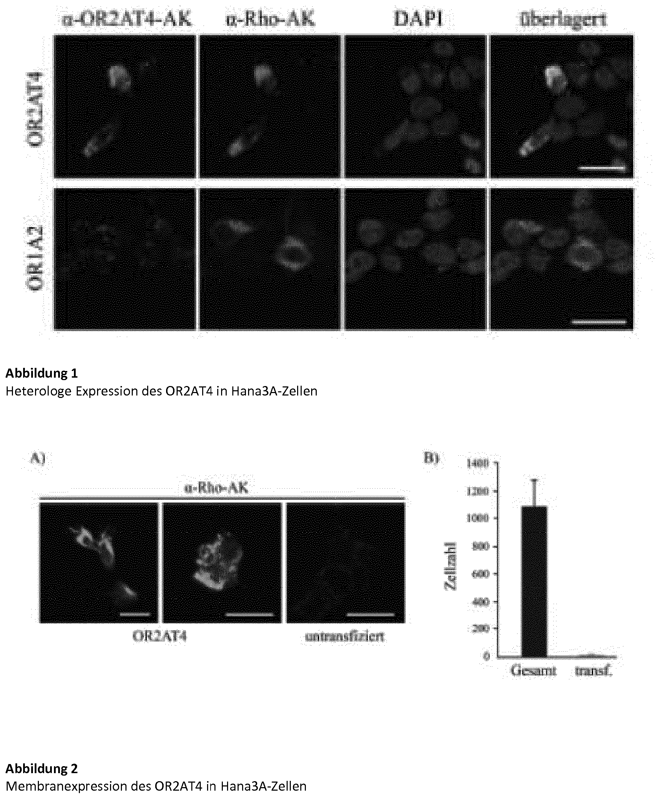

Zum Nachweis der heterologen Expression des OR2AT4 in Hana3A-Zellen wurden diese mit dem Rezeptor-Plasmid (pcDNA3-OR2AT4) für 48 Stunden mit der Calcium-Phosphat- Methode transfiziert und immuncytochemische Färbungen mit einem OR2AT4-spezifischen (α-OR2AT4-AK) und Rhodopsin-spezifischen Antikörper (α-Rho-AK) durchgeführt. Durch die deutlich sichtbare Co-Färbung der beiden Antikörper konnte gezeigt werden, dass der α-OR2AT4-AK den Rezeptor spezifisch bindet und eine heterologe Expression des Rezeptors in Hana3A-Zellen möglich ist. N-Terminus der codierenden Sequenz des ORs angefügt, wodurch die heterologe Expression ebenfalls unterstützt werden soll. Zum Nachweis der heterologen Expression des OR2AT4 in Hana3A-Zellen wurden diese mit dem Rezeptor-Plasmid (pcDNA3-OR2AT4) für 48 Stunden mit der Calcium-Phosphat-Methode transfiziert und immuncytochemische Färbungen mit einem OR2AT4-spezifischen (α -OR2AT4-AK) und Rhodopsin-spezifischen Antikörper (α -Rho-AK) durchgeführt. Durch die deutlich sichtbare Kofärbung der beiden Antikörper konnte gezeigt werden, dass der α-OR2AT4-AK den Rezeptor spezifisch bindet und eine heterologe Expression des Rezeptors in Hana3A-Zellen möglich ist **(****Abbildung 1****, oben).**

Die unspezifische Bindung des α-OR2AT4-AKs konnte durch Kontrollfärbungen mit einem anderen heterolog exprimierbaren OR, dem OR1A2, ausgeschlossen werden. In Hana3AZellen, transfiziert mit dem OR1A2, zeigte sich durch eine deutliche Rhodopsin-Färbung die Expression des Rezeptors, eine Färbung des α-OR2AT4-AKs war jedoch nicht erkennbar **(****Abbildung 1****, unten).** Als weitere Kontrolle wurden Hana3A-Zellen ohne Primärantikörper inkubiert, um eine unspezifische Bindung des Zweitantikörpers auszuschließen.

### Membranexpression des OR2AT4 in Hana3A-Zellen

Damit die Bindung von extrazellulären Liganden an den heterolog in Hana3A-Zellen exprimierten OR2AT4 gewährleistet ist, muss dieser in die Plasmamembran eingebaut werden. Für OR ist bekannt, dass sie sich im heterologen System nur sehr schwer exprimieren lassen, was oft auf einem fehlenden Transport der Rezeptoren vom endoplasmatischen Retikulum zur Zelloberfläche basiert. Es ist daher essentiell, den Einbau des Rezeptors in die Membran vor der Durchführung von Deorphanisierungsstudien zu überprüfen sowie die Transfektionsrate zu bestimmen. Hierfür wurde eine Lebendzellfärbung nach Zhang & Matsunami (2008) durchgeführt, bei der selektiv nur Oberflächenproteine angefärbt werden können, da die Zellmembran nicht permeabilisiert wird.

Für die Färbung wurde erneut der α-Rho-AK verwendet, der den N-Terminal liegenden Rho*tag* des rekombinant exprimierten ORs detektiert. Die Färbung zeigte, dass nach 48-stündiger Transfektion mit der Calcium-Phosphat-Methode der OR2AT4 erfolgreich in die Membran eingebaut wird **(****Abbildung 2a****).** Durch die Bestimmung der Anzahl gefärbter Hana3AZellen (MW = 12 Zellen) relativ zur Gesamtzellzahl (MW = 1050 Zellen), ließ sich eine Transfektionsrate von ca. 1,3 % ermitteln **(****Abbildung 2b****).**

Zusammenfassend zeigen die vorliegenden Ergebnisse, dass der OR2AT4 in Hana3A-Zellen membranständig exprimiert wird und die Voraussetzungen für die Charakterisierung des rezeptiven Feldes des Rezeptors daher gegeben waren.

### Das rezeptive Feld des rekombinant exprimierten OR2AT4

Eine am Lehrstuhl für Zellphysiologie der Ruhruniversität Bochum etablierte Methode für Deorphanisierungsstudien ist das *Calcium Imaging,* bei der die Aktivierung eines ORs durch einen Duftstoff über eine Veränderung der intrazellulären Calciumkonzentration der transfizierten Zellen mit Hilfe eines fluoreszierenden Farbstoffes sichtbar gemacht wird [vgl. Wetzel et al. in: J. Neurosci. 19, S. 7426-7433 (1999**);** Spehr et al. in: Science 299, S. 2054-2058 (2003**);** Neuhaus et al. in: J. Biol. Chem. 284, S. 16218-16225 (2009)].Bei dieser Methode werden die Hana3A-Zellen kurzzeitig (20 s) mit einem Duftstoff stimuliert und für Duftstoffscreenings hohe Konzentrationen (bis zu 1 mM) gewählt, um eine Aktivierung von in Hana3A-Zellen gering exprimierten OR sicherzustellen.

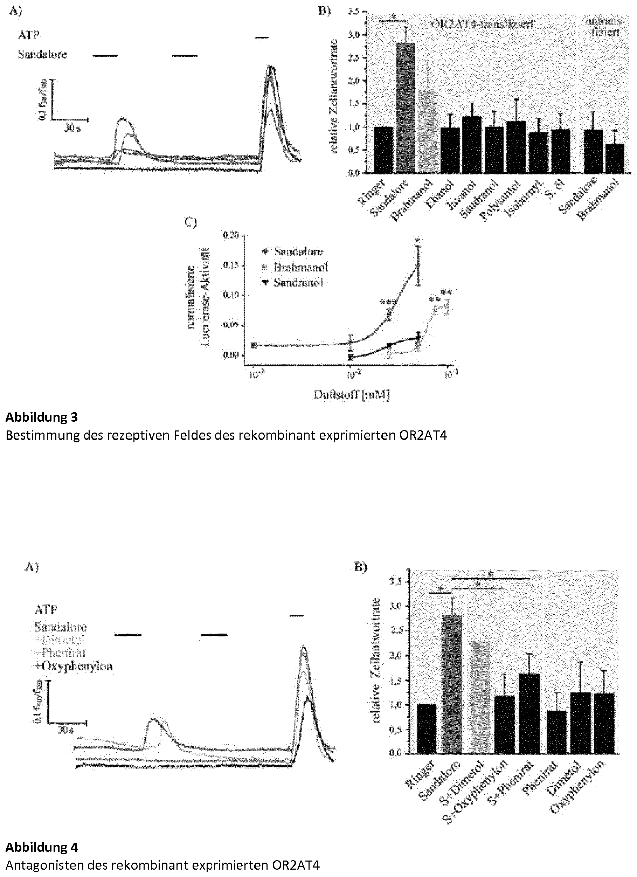

**Untersuchung potenzieller Agonisten des rekombinant exprimierten OR2AT4.** Die Aktivierung des OR2AT4 durch Sandalore^{®} wurde zunächst mittels der *Calcium Imaging-*Methode überprüft. Hier zeigten transient OR2AT4-transfizierte Hana3A-Zellen bei Stimulation mit 1 mM Sandalore^{®} spezifische Calciumsignale, wobei die Zellen nur einmal durch den Sandelholzduft aktivierbar waren **(****Abbildung 3a****).** Bei der Quantifizierung ergab sich eine signifikant erhöhte Antwortrate der Hana3A-Zellen relativ zur Ringer-Kontrolle, was die Aktivierung des Rezeptors durch den Sandelholzduft bestätigte. Da ein Rezeptor oft von mehreren strukturverwandten Molekülen aktiviert werden kann wurde der OR2AT4 mit sechs weiteren synthetischen Sandelholzdüften (Brahmanol, Ebanol, Isobornylcyclohexanol, Javanol, Polysantol und Sandranol) sowie mit natürlichem Sandelholzöl stimuliert und die Aktivierung des Rezeptors überprüft. Hierbei zeigte nur Brahmanol eine erhöhte, jedoch nicht signifikante, Antwortrate in *Calcium Imaging*-Experimenten **(****Abbildung 3b****)**

Durch Kontrollmessungen von untransfizierten Hana3A-Zellen konnte ausgeschlossen werden, dass die erhöhte Antwortrate der beiden Sandelholzdüfte durch die direkte Aktivierung der Hana3A-Zellen hervorgerufen wird **(****Abbildung 3b****, rechts).**

Zur Verifizierung der *Calcium Imaging*-Ergebnisse und zur Untersuchung einer dosisabhängigen Aktivierung des OR2AT4 wurde eine weitere Methode der Deorphanisierung, basierend auf einem dualen Luciferase-System, angewandt. Hierbei werden zusätzlich zum OR zwei Luciferasen in Hana3A-Zellen eingebracht. Wenn der exprimierte OR durch einen Duftstoff aktiviert und im weiteren Verlauf der Signalkaskade cAMP gebildet wird, initiiert dies über CRE (*cAMP response element*) die Expression der *Firefly*-Luciferase. Die zweite Luciferase, die *Renilla*-Luciferase, dient als Kontrolle für Transfektion und Vitalität der Zellen. Durch Verrechnung der beiden Luciferase-Werte kann dann eine Aussage über die Aktivierbarkeit eines ORs durch einen Duftstoff getroffen werden. Im CRE-Luciferase-Assay verbleibt der Duftstoff für 4 Stunden auf den Zellen, um die Expression der Luciferase zu gewährleisten. Durch die längere Inkubationszeit der Duftstoffe und die Möglichkeit mehr Rezeptoren in einem 96-Well-Format zu aktivieren, ist diese Methode sensitiver als das *Calcium Imaging* und eignet sich gut für die Erstellung von Dosis-Wirkungs-Beziehungen.

Im durchgeführten CRE-Luciferase-Assay zeigten Sandalore^{®} und Brahmanol^{®} eine signifikante, dosisabhängige Aktivierung des rekombinant in Hana3A-Zellen exprimierten Rezeptors. Für Brahmanol wurde jedoch eine höhere Konzentration (≥75 µM) als bei Sandalore^{®} (≥ 25 µM) benötigt, um einen signifikanten Effekt am Rezeptor hervorzurufen **(****Abbildung 3c****).** Als weiterer Sandelholzduft wurde Sandranol^{®} verwendet, der in der untersuchten Konzentration (≤ 50 µM) hingegen keine Reaktion zeigte **(****Abbildung 3c****).**

Da die Duftstoffe durch die 4-stündige Inkubation im Luciferase-Assay ab einer bestimmten Konzentration toxisch auf Hana3A-Zellen wirkten, konnten keine höheren Duftstoffkonzentrationen (Sandalore^{®} ≤ 50 µM, Brahmanol^{®} ≤ 100 µM, Sandranol^{®} ≤ 50 µM) verwendet und somit keine vollständigen Dosis-Wirkungs-Kurven erstellt werden. Diese Toxizität zeigte sich unter anderem in reduzierten Werten des Kontrollreporters *Renilla-*Luciferase. Daher wurden zur Auswertung nur Werte mit einheitlich hohen *Renilla*-Signalen herangezogen (Zhuang und Matsunami, 2008). Für die Sandelholzdüfte Javanol, Polysantol, Isobornylcyclohexanol und das Sandelholzöl lagen die höchsten verwendbaren Konzentrationen bei 10-25 µM. Bis zu dieser Konzentration zeigte sich jedoch keine erhöhte Aktivierung des OR2AT4.

**Untersuchung potenzieller Antagonisten des rekombinant exprimierten OR2AT4.** Duftstoffe können einen Rezeptor nicht nur aktivieren, sondern auch als Antagonisten für einen OR wirken. Mittels der *Calcium Imaging*-Technik wurden die Duftstoffe Oxyphenylon und Phenirat als Antagonisten identifiziert, welche die Sandalore^{®}-induzierten Calciumsignale bei Koapplikation mit Sandalore^{®} (1:1, jeweils 1 mM) signifikant reduzierten. Als Kontrolle wurde der Duftstoff Dimetol verwendet, der bei gemeinsamer Applikation keinen Effekt auf die Sandalore^{®}-induzierten Calciumsignale hatte. Dimetol, Phenirat und Oxyphenylon alleine zeigten keine Reaktion auf die Hana3A-Zellen.

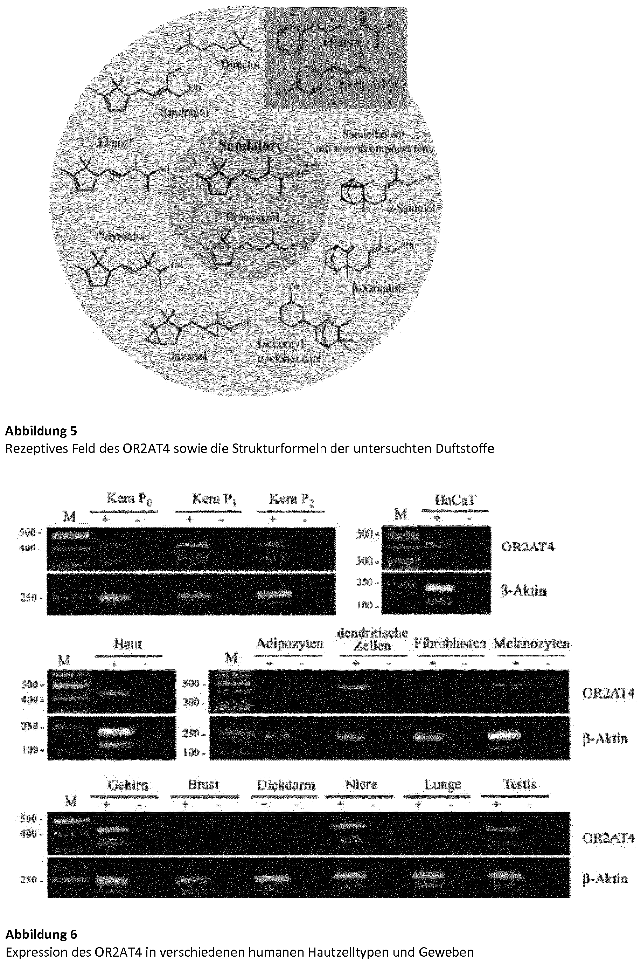

Zusammenfassend ergaben sich für den heterolog exprimierten OR2AT4 Sandalore^{®} und Brahmanol^{®} als Agonisten sowie Oxyphenylon und Phenirat als Antagonisten. In **Abbildung 5** sind das rezeptive Feld des OR2AT4 sowie die Strukturformeln der untersuchten Duftstoffe graphisch dargestellt.

### EXPRESSIONSNACHWEIS DES OR2AT4 IN HUMANEN KERATINOZYTEN

Zur Identifikation der ektopischen Expression des OR2AT4 in verschiedenen humanen Hautzelltypen und Geweben wurden RT-PCR-Analysen durchgeführt. Darüber hinaus wurden die Expressionsanalysen des OR2AT4 in humanen Keratinozyten durch immuncytochemische Färbungen erweitert.

### Expression des OR2AT4 in humanen Hautzellen und Geweben mittels RT-PCR

Für den Nachweis des OR2AT4 auf Transkriptebene wurde RNA von verschiedenen Hautzelltypen und Hautbiopsaten isoliert und eine RT-PCR durchgeführt. Transkripte des OR2AT4 konnten in humanen primären Keratinozyten verschiedener Differenzierungsstadien, in HaCaT-Zellen und in humanen Hautbiopsaten (Vollhaut) nachgewiesen **werden (****Abbildung 6****).** Da menschliche Haut neben dem Hauptbestandteil der Keratinozyten aus weiteren Zellen wie Bindegewebs-, Pigment- und Immunzellen besteht, wurden die Zelltypen einzeln per RT-PCR auf die Expression des Rezeptors überprüft. Es zeigte sich, dass dendritische Zellen und Melanozyten ebenfalls den OR2AT4 exprimieren. Dahingegen konnte in Adipozyten und Fibroblasten keine Expression des Rezeptors nachgewiesen werden **(****Abbildung 6****, Mitte).**

Um herauszufinden, ob der Rezeptor exklusiv in der Haut exprimiert vorliegt, wurden Gesamt-RNA-Proben von verschiedenen menschlichen Geweben (Gehirn, Brust, Dickdarm, Niere, Lunge und Testis) für weitere RT-PCR-Analysen verwendet. Hier zeigte sich, dass der OR2AT4 nicht ausschließlich in der Haut, sondern auch in anderen Geweben wie Gehirn, Niere und Testis exprimiert wird. In Brust, Dickdarm und Lunge wurden hingegen keine Transkripte nachgewiesen **(****Abbildung 6****, unten).** Um auszuschließen, dass PCR-Produkte aufgrund von schlecht isolierter oder degradierter RNA ausblieben, wurde zur Überprüfung der RNA-Qualität für alle Proben eine β-Aktin-Kontrolle durchgeführt, die für alle RNA-Proben positiv war **(****Abbildung 6****).**

### Expression des OR2AT4 in humanen Keratinozyten mittels Immuncytochemie

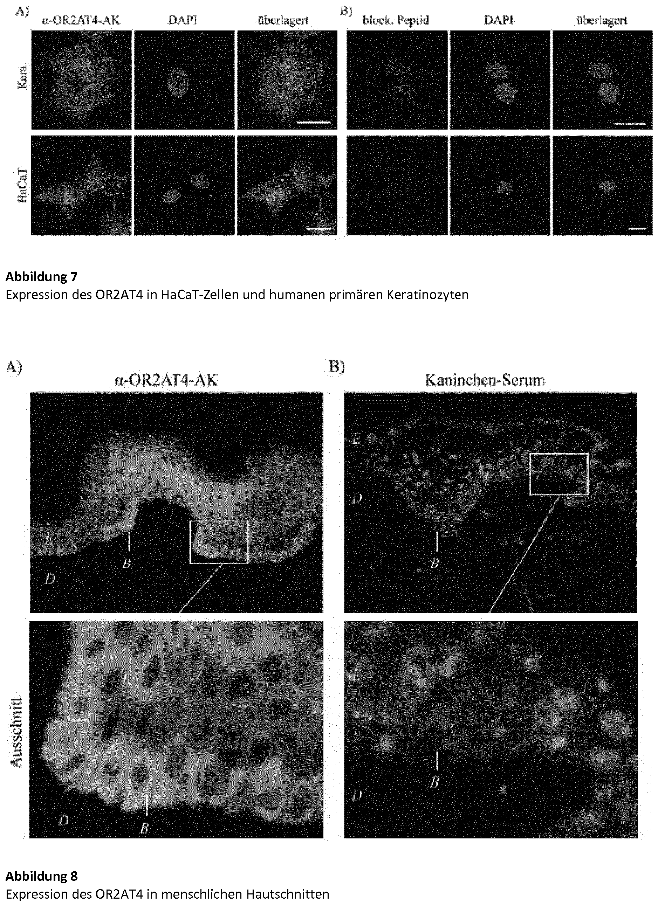

Zum Nachweis der Expression des OR2AT4 auf Proteinebene wurden immuncytochemische Färbungen mit einem selbst generierten α-OR2AT4-AK durchgeführt. Die Spezifität des Antikörpers wurde an Hana3A-Zellen, die transient mit OR2AT4 transfiziert wurden, überprüft. Bei Verwendung des α-OR2AT4-AK war eine deutliche Färbung der HaCaT-Zellen sowie der Keratinozyten in den konfokalen Immunfluoreszenzbildern zu erkennen **(****Abbildung 7a****)**

Um die unspezifische Bindung des Antikörpers an Hautzellen auszuschließen, wurde ein blockierendes Peptid verwendet. Dieses blockiert den α-OR2AT4-AK, wodurch er sich nicht mehr an die Bindestelle anlagern kann. Bei Verwendung des blockierenden Peptids war nur noch eine leichte Färbung des Zellkerns und der Bereiche um den Zellkern erkennbar **(****Abbildung 7b****).** Als weitere Kontrolle wurden die Zellen ohne Primärantikörper inkubiert, um eine unspezifische Bindung des Zweitantikörpers auszuschließen (Daten nicht gezeigt). Die Bindung des α-OR2AT4-AKs ist somit spezifisch und die Expression des Rezeptors auf Proteinebene in humanen Keratinozyten konnte nachgewiesen werden.

Zusätzlich zu kultivierten Keratinozyten wurde die Proteinexpression des OR2AT4 in der menschlichen Haut untersucht. Dazu wurden Hautschnitte ebenfalls mit dem α-OR2AT4-AK gefärbt. In den Färbungen war eine deutliche Expression des Rezeptors in epidermalen Keratinozyten erkennbar **(****Abbildung 8a****, oben),** wobei basale Keratinozyten die stärkste Bindung des Antikörpers aufwiesen **(****Abbildung 8a****, Ausschnitt).** Die unter der Epidemis lokalisierte Dermis zeigte keine spezifische Färbung. Auch Kontroll-Färbungen, in denen der Primärantikörper durch Kaninchen-Serum ersetzt wurde, wiesen keine spezifische Färbung auf **(****Abbildung 8b****).**

### Charakterisierung der Sandalore^{®}-induzierten Calciumsignale in humanen Keratinozyten

Zur Charakterisierung der physiologischen Funktion des OR2AT4 in Hautzellen ist es wichtig, die Aktivierbarkeit dieses Rezeptors durch die identifizierten Liganden Sandalore^{®} und Brahmanol^{®} in humanen, kultivierten Keratinozyten zu untersuchen. In diesem Teil der Arbeit lag der Fokus auf dem Liganden Sandalore^{®}.

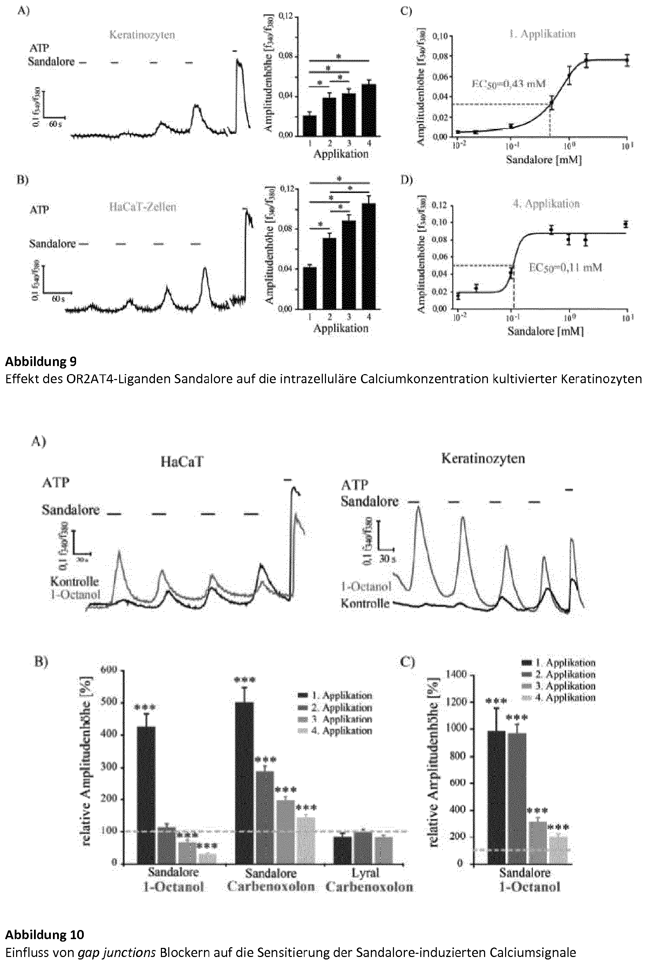

In *Calcium Imaging*-Experimenten werden 80-90 % der HaCaT-Zellen sowie der primären Keratinozyten repetitiv durch Sandalore^{®} stimuliert, wobei die Sandalore-induzierten Calciumsignale eine signifikante Sensitisierung, also eine Zunahme der Signalamplitude nach jeder weiteren Applikation zeigten **(Abbildungen 9a und 9b).**

Die genauere Untersuchung der Amplituden der ersten Applikation zeigte eine dosisabhängige Aktivierung der HaCaT-Zellen durch Sandalore^{®}, die bei 100 µM beginnt und ab ca. 2 mM gesättigt ist **(****Abbildung 9c****).** Bei der 4. Applikation trat die Sättigung schon bei ca. 500 µM ein **(****Abbildung 9d****).** Der ECR50R-Wert liegt bei der 1. Applikation bei 430 µM Sandalore^{®} und bei der 4. Applikation bei 112 µM Sandalore^{®}.

### Untersuchung der Sensitisierung der Sandalore^{®}-induzierten Calciumsignale

Für die Sandalore^{®}-induzierten Calciumsignale ist die Zunahme der Amplituden mit repetitiver Stimulation charakteristisch **(****Abbildung 9****).** Um die Beteiligung von Zell-Zell-Kanälen (*gapjunctions*), über die Keratinozyten Moleküle wie ATP oder Ca²⁺ austauschen können, an diesem Sensitisierungsmechanismus zu untersuchen, wurden die zwei *gap junction-*Blocker 1-Octanol und Carbenoxolon verwendet.

Nach Vorinkubation von Keratinozyten oder HaCaT-Zellen mit dem Blocker 1-Octanol war eine Verstärkung der Sandalore^{®}-induzierten Calciumsignale in *Calcium Imaging*-Experimenten erkennbar **(****Abbildung 10a****).** Die Quantifizierung der Ergebnisse zeigte, dass die erste durch Sandalore^{®} induzierte Amplitude bei Verwendung von 1-Octanol bei HaCaT-Zellen um ca. 400 % **(****Abbildung 10b****)** und bei Keratinozyten sogar um fast 1000 % **(****Abbildung 10c****)** erhöht war. Die folgenden Calciumsignale nahmen bei repetitiver Sandalore^{®}-Stimulation in Relation zu den Kontrollmessungen immer weiter ab. Die Verwendung des zweiten *gap junction-*Blockers Carbenoxolon führte zum gleichen Ergebnis **(****Abbildung 10b****)**

Um ausschließen zu können, dass unspezifische Effekte durch das Blockieren der *gap junctions* auftraten, wurden die Zellen ebenfalls nach Vorinkubation mit Carbenoxolon mit Lyral, einem weiteren Duftstoff, der Calciumsignale in Hautzellen induziert, stimuliert. Hier zeigte sich kein Effekt auf das Lyral-induzierte Antwortmuster **(****Abbildung 10b****)**

### Pharmakologische Charakterisierung der Sandalore^{®}-induzierten Calciumsignale

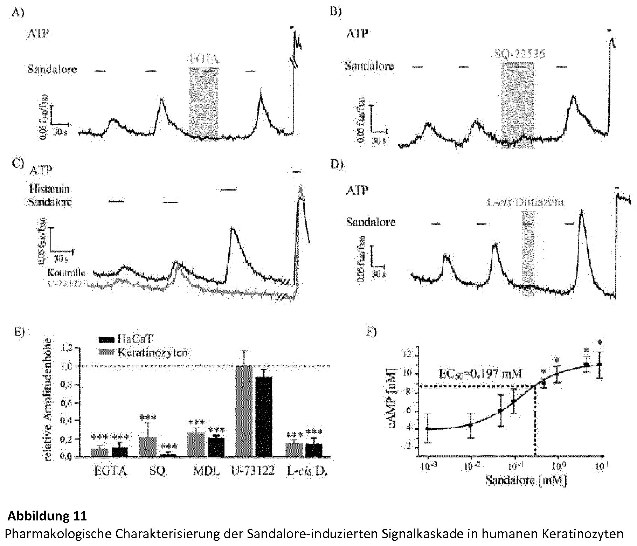

Mittels der *Calcium Imaging*-Technik und der Verwendung von spezifischen Blockern für Signalkaskadenproteine wurde die Sandalore^{®}-induzierte Signalkaskade für HaCaT-Zellen und primären Keratinozyten pharmakologisch charakterisiert. Zunächst wurde untersucht, ob die Calciumsignale durch einen Calciumeinstrom aus dem extrazellulären Raum entstehen. Dafür wurde calciumfreie extrazelluläre Lösung verwendet, der ein Calciumchelator (EGTA) zugesetzt wurde. In Gegenwart der calciumfreien Lösung waren die Sandalore^{®}-induzierten Calciumsignale signifikant reduziert **(Abbildungen 11a und 11e).** Zur Untersuchung der Beteiligung einer Adenylylcyclase wurden die spezifischen Blocker SQ-22536 und MDL-12330A verwendet. Auch hier zeigte sich eine signifikante Reduktion der Sandalore-induzierten Calciumsignale in Gegenwart des jeweiligen Blockers (Abbildung 11b und 11e). Die Beteiligung der Phospholipase C konnte hingegen ausgeschlossen werden, da der PLC-Blocker U-73122 keinen Einfluss auf die Sandalore^{®}-induzierten Calciumsignale zeigte (Abbildung 11c und 11e). Die Wirksamkeit des Blockers wurde hier mit Histamin überprüft, was den PLC-Signalweg aktiviert **(****Abbildung 11c****).**

Zur Identifikation des Calciumkanals, über den die Calciumionen von außen in das Cytosol der Hautzellen strömen, wurde der CNG-Kanal-Blocker *L-cis* Diltiazem verwendet. Auch dieser reduzierte signifikant die Sandalore^{®}-induzierten Calciumsignale (Abbildung 11d und 11e). Zur Verifizierung der Daten der pharmakologischen Charakterisierung mittels der *Calcium Imaging*-Technik wurde zusätzlich ein cAMP-Assay durchgeführt. Hierzu wurden HaCaTZellen mit unterschiedlichen Sandalore^{®}-Konzentrationen stimuliert und der cAMP-Gehalt bestimmt. Es ergab sich eine dosisabhängige cAMP-Erhöhung mit einem EC50-Wert von 197 µM Sandalore^{®} **(****Abbildung 11f****).**

Zusammenfassend ergab sich somit für die Sandalore^{®}-induzierten Calciumsignale der HaCaTZellen und primären Keratinozyten ein cAMP-abhängiger Signalweg mit Beteiligung eines CNG-Kanals.

### Expression von olfaktorischen Signalkaskadenkomponenten und strukturverwandten Proteinen

Für Spermien und die Niere wurden neben der funktionalen ektopischen Expression von OR zusätzlich Komponenten der olfaktorischen Signalkaskade, wie die Untereinheit des olfaktorischen G-Proteins G·olf und die Adenylylcyclase 3 (AC3) nachgewiesen. Darüber hinaus zeigen Studien neben der ektopischen Expression von OR, die weite Verbreitung von Komponenten der olfaktorischen Signalkaskade in den verschiedensten Geweben Im Rahmen dieser Arbeit wurde daher mit Hilfe einer Transkriptom-Analyse (*Next Generation Sequencing,* NGS) ein Überblick über die in Keratinozyten exprimierten, an die olfaktorische Signalkaskade angelehnten Signalkaskadenproteine geschaffen.

**Expression von olfaktorischen Signalkaskadenkomponenten und strukturverwandten Proteinen auf Transkriptebene.** Für die Untersuchung der Signalkaskadenproteine wurden NGS-Daten von primären Keratinozyten ausgewertet. Neben den in der olfaktorischen Signalkaskade vorkommenden Komponenten wie Gαolf, AC3, CNGA2 und TMEM16B wurden weitere strukturverwandte Signalkaskadenproteine untersucht. Durch die Auswertung der Transkriptom-Daten der primären Keratinozyten, konnte die Expression der Untereinheit des Gαolf (FPKM=3,6) und der AC3 (FPKM=2,1) bestätigt werden. Die Expression fiel im Vergleich zu verwandten Proteinen wie der Untereinheit des stimulatorischen G-Proteins Gas (FPKM=69,2) oder der AC6 (FPKM=14,1) eher gering aus.

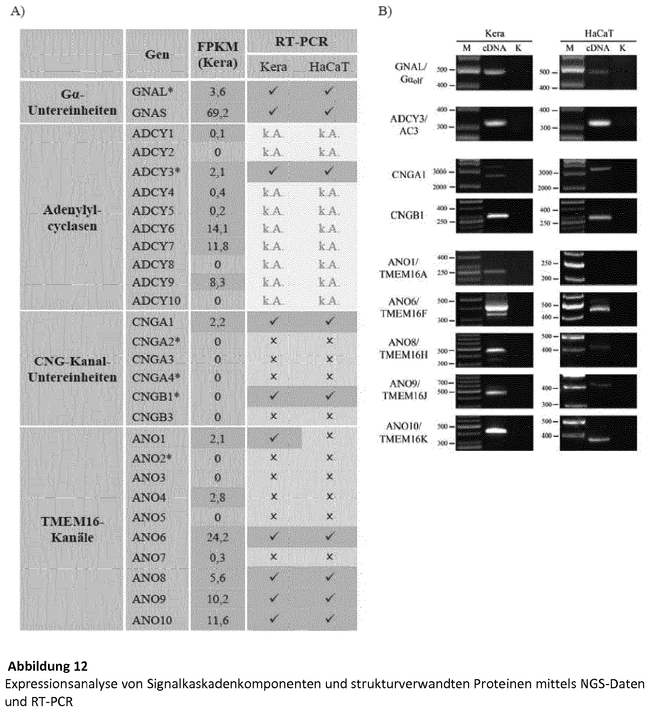

Weiterhin wurden Transkripte der CNG-Kanal-Untereinheit CNGA1 (FPKM=2,2) sowie sieben der zehn untersuchten Vertreter der Chloridkanal-Familie TMEM16 in Keratinozyten nachgewiesen **(****Abbildung 12a****).** Zur Validierung der NGS-Daten wurden RT-PCR Experimente mit primären Keratinozyten und HaCaT-Zellen durchgeführt (Abbildung 12b). Für beide Zelltypen konnte die Expression von Gαolf und AC3 bestätigt werden. Weiterhin konnte die Expression der Chloridkanäle TMEM16H, TMEM16J, TMEM16F, TMEM16K sowie der CNGA1-Untereinheit neben primären Keratinozyten auch in HaCaT-Zellen nachgewiesen werden. Die hier verwendeten NGS-Daten weisen eine geringe Sequenziertiefe auf (^{~}18 Millionen *reads*), so dass gering exprimierte Gene mit dieser Methode möglicherweise nicht nachgewiesen werden konnten. Daher wurden einige der laut NGS-Daten nicht exprimierten Gene ebenfalls per RT-PCR untersucht. Hier zeigte sich, dass neben der CNGA1-Untereinheit auch die CNGB1-Untereinheit in HaCaT-Zellen und Keratinozyten exprimiert wird.

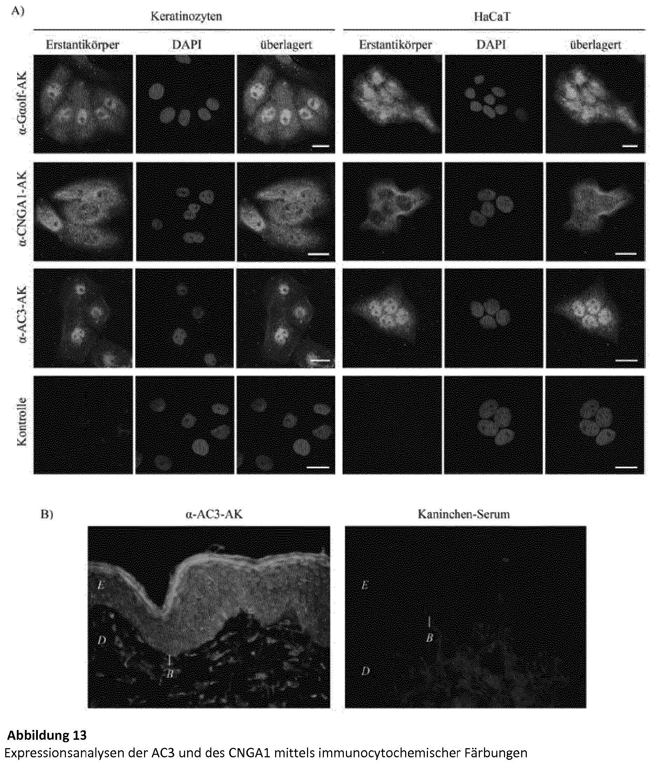

**Expression von Komponenten der olfaktorischen Signalkaskade und strukturverwandten Proteinen auf Proteinebene.** Neben RT-PCR-Experimenten wurden immuncytochemische Färbungen zum Nachweis der Komponenten der olfaktorischen Signalkaskade G·olf, RAC3 und CNGA1 mit HaCaT-Zellen und primären Keratinozyten angefertigt. Für beide Zelltypen war ein deutliche Expression des Gαolf, der AC3 und des CNGA1 in den immuncytochemischen Färbungen zu erkennen (Abbildung 13a). Die Gαolf- und AC3-Färbung zeigten neben einer cytosolischen Lokalisation auch eine deutliche Zellkernfärbung. Färbungen ohne Primärantikörper dienten als Kontrolle und wiesen keine unspezifische Bindung des Sekundärantikörpers auf.

Zusätzlich zu den kultivierten Keratinozyten erfolgte im Vorfeld ein Nachweis des Gαolf über immuncytochemische Färbungen in Hautschnitten. Zur Vervollständigung der Daten wurden humane Hautschnitte mit dem α-AC3-AK gefärbt. Hier war eine starke Expression der AC3 in epidermalen Keratinozyten zu erkennen. Kontroll-Färbungen, in denen der Primärantikörper durch Kaninchen-Serum ersetzt wurde, wiesen keine spezifische Färbung auf **(****Abbildung 13b****).**

### BETEILIGUNG DES OR2AT4 AN SANDALORE^{®}-INDUZIERTEN CALCIUMSIGNALEN IN HUMANEN KERATINOZYTEN

Humane Keratinozyten exprimieren den Rezeptor OR2AT4 und zeigen Calciumsignale bei Stimulation mit dem Liganden Sandalore^{®}. Zur Überprüfung, dass die Sandalore^{®}-induzierten Calciumsignale über den OR2AT4 und nicht über einen anderen Rezeptor bzw. Mechanismus vermittelt werden, sollten zum einen RNA Interferenz-Versuche und zum anderen die vorstehend beschriebenen Antagonisten des OR2AT4 getestet werden.

### RNA-Interferenz-Versuche

Bei der RNA-Interferenz wird eine *small interfering* RNA (siRNA) in eine Zelle eingebracht, wodurch2T die mRNA, welche die komplementäre1T2T 1T2TNucleotidsequenz der siRNA besitzt, abgebaut und das entsprechende codierte1T2T 1TProtein1T somit 1T2Tnicht mehr synthetisiert wird. Für die hier durchgeführten RNA-Interferenz-Versuche wurden zwei selbst generierte siRNA verwendet, die gegen den OR2AT4 gerichtet sind. Bei erfolgreicher Wirkung der siRNA wird die mRNA des OR2AT4 abgebaut, wodurch kein funktionsfähiges Rezeptorprotein translatiert und folglich die Sandalore^{®}-induzierten Calciumsignale reduziert sein sollten.

Um unspezifische Effekte ausschließen zu können, wurden zwei Kontroll-siRNA (scRNA) verwendet, die keinen Effekt auf die Zellen zeigen sollten. Zunächst wurde in einem Kontrollexperiment an Hana3A-Zellen überprüft, ob die beiden selbst generierten siRNA erfolgreich zum Abbau der Transkripte des OR2AT4 führen. Hierzu wurden Hana3A-Zellen mit einem speziellen Luciferase- und OR2AT4-exprimierenden Reporter-Plasmid (pmirGLO-OR2AT4) und den jeweiligen siRNA oder scRNA kotransfiziert. Wird die mRNA des OR2AT4 durch die siRNA abgebaut, wird aufgrund einer Fusions-mRNA aus Luciferase und OR2AT4 die mRNA der Luciferase zerstört und folglich das Lumineszenzsignal reduziert.

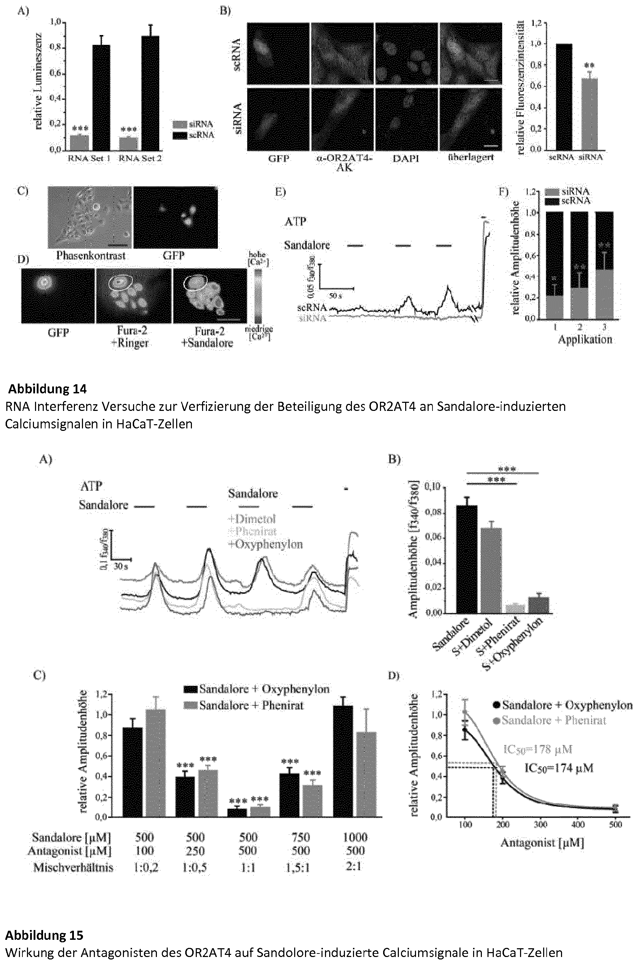

Die Ergebnisse zeigten, dass die beiden siRNA-Varianten zum Abbau der Transkripte des OR2AT4 führen, was durch die signifikante Reduktion des Lumineszenzsignals deutlich wird. Die beiden scRNA zeigten keinen Effekt **(****Abbildung 14a****).** Die Voraussetzungen für erfolgreiche *knockdown*-Experimente waren demnach gegeben und die Funktionalitätsversuche konnten an HaCaT-Zellen durchgeführt werden. Vorab wurde überprüft, ob die Expression des OR2AT4 durch die siRNA auch in HaCaTZellen reduziert ist. Hierzu wurden HaCaT-Zellen zwei Tage mit einer Mischung aus den beiden siRNA oder scRNA transfiziert und immuncytochemische Färbungen, in denen der spezifische OR2AT4-Antikörper verwendet wurde, durchgeführt. HaCaT-Zellen, die erfolgreich die siRNA scRNA aufgenommen hatten, konnten mit Hilfe von GFP, welches über den pGeneClip-Vektor ebenfalls exprimiert wird, identifiziert werden. Der Vergleich der α-OR2AT4-AK-Färbungen von siRNA- oder scRNA-exprimierenden Zellen zeigte, dass die Färbung und damit die Expression des OR2AT4 bei Verwendung der siRNA signifikant reduziert war und die siRNA somit auch in HaCaT-Zellen funktional ist **(****Abbildung 14b****).**

Um festzustellen, ob der OR2AT4 an den Sandalore^{®}-induzierten Calciumsignalen beteiligt ist, wurden HaCaT-Zellen für zwei Tage mit einer Mischung aus den zwei siRNA oder scRNA transfiziert **(****Abbildung 14c****)** und in *Calcium Imaging*-Versuchen verwendet. Während der Messungen konnte zwischen siRNA- oder scRNA-exprimierenden und nicht exprimierenden Zellen durch die Koexpression von GFP unterschieden werden **(****Abbildung 14d****).** Die transfizierten Zellen wurden repetitiv mit Sandalore^{®} stimuliert und die Amplituden der Calciumsignale berechnet. Die HaCaT-Zellen, transfiziert mit siRNA, zeigten eine signifikante Reduktion der Sandalore^{®}-induzierten Calciumsignale relativ zu den scRNA-exprimierenden Zellen für alle drei Sandalore^{®}-Applikationen **(Abbildungen 14e und 14f).**

### Wirkung der Antagonisten auf Sandalore^{®}-induzierte Calciumsignale in humanen Keratinozyten

Mit Hilfe von Antagonisten kann die Aktivierung eines Rezeptors durch seine spezifischen Liganden ebenfalls verifiziert werden, da die Inhibition des Liganden-induzierten Calciumsignals im heterologen System und in den nativen Zellen für die Aktivierung des gleichen Rezeptors spricht. Es wurde bereits beschrieben, dass Phenirat und Oxyphenylon für den OR2AT4 als Antagonisten identifiziert wurden, die jeweils bei gemeinsamer Applikation die Sandalore^{®}-induzierten Calciumsignale im heterologen System inhibierten. Als Kontrolle wurde Dimetol verwendet, welches bei Koapplikation mit Sandalore keinen inhibitorischen Effekt zeigte. Die beiden Antagonisten wurden daher auf eine potenzielle Inhibition der Sandalore^{®}-induzierten Calciumsignale in HaCaT-Zellen getestet, um eine Beteiligung des OR2AT4 zu überprüfen.

Zur Charakterisierung der Antagonisten wurden HaCaT-Zellen zunächst repetitiv mit Sandalore^{®} (1 mM) stimuliert. Bei der dritten Applikation erfolgte eine Kostimulation (1:1) von Sandalore und einem der Antagonisten. Bei Koapplikation von Phenirat bzw. Oxyphenylon war eine signifikante Reduktion der Sandalore-induzierten Calciumsignale erkennbar, wohingegen bei der Koapplikation mit Dimetol die Calciumsignale nur leicht reduziert waren **(Abbildungen 15a und 15b)** Darauf aufbauend sollte eine dosisabhängige Charakterisierung der Inhibition erfolgen. Hierzu wurden Sandalore und die Antagonisten mit unterschiedlichen Konzentrationen kostimuliert. Bei gemeinsamer Applikation von Sandalore (500 µM) mit Oxyphenylon (500 µM) oder Phenirat (500 µM) waren die Calciumsignale signifikant auf 8 % bzw. 9 % der Kontrollamplitude (Sandalore^{®} ohne Antagonisten) reduziert. Bei Reduktion der Antagonisten-Konzentration auf 250 µM verringerte sich die Inhibition der Sandalore^{®}-induzierten Calciumsignale und die Amplitudenhöhen lagen bei 39 % bzw. 45 % der Kontrollamplituden. Bei weiterer Reduktion der Antagonisten-Konzentration auf 100 µM wurde die inhibitorische Wirkung beider Antagonisten komplett aufgehoben **(****Abbildung 15c****).** Der ermittelte IC50-Wert (mittlere inhibitorische Konzentration) für Phenirat lag bei 178 µM und für Oxyphenylon bei 174 µM **(****Abbildung 15d****).**

Bei konstanter Antagonisten-Konzentration (500 µM) und gleichzeitiger Erhöhung der Sandalore^{®}-Konzentration (750 µM), verringerte sich der Block durch die Antagonisten ebenfalls. Bei weiterer Erhöhung auf 1 mM Sandalore^{®} wurde die Inhibition durch die Antagonisten komplett aufgehoben **(****Abbildung 15c****).** Insgesamt zeigte sich eine dosisabhängige Inhibition der Sandalore^{®}-induzierten Calciumsignale durch die Antagonisten Oxyphenylon und Phenirat. Dies stützt die Ergebnisse der siRNAVersuche und bestätigt die Aktivierung des OR2AT4 durch den Liganden Sandalore^{®} in HaCaTZellen.

### PHYSIOLOGISCHE FUNKTION DES OR2AT4 IN HUMANEN KERATINOZYTEN

In *Calcium Imaging*-Experimenten wurden bei kurzzeitigen Simulationen (20 s) mit Sandalore^{®} Änderungen der intrazellulären Calciumkonzentration in Hautzellen induziert. Bei der regelmäßigen Verwendung von Cremes oder Parfüms verbleiben Duftstoffe jedoch für einen längeren Zeitraum (Stunden bis Tage) auf der Haut. Physiologische Effekte, die Sandalore^{®} hierbei durch die Aktivierung des OR2AT4 in Hautzellen auslösen kann, wurden mit Langzeitstimulationsexperimenten untersucht.

### Auswirkung der Sandalore^{®}-Stimulation auf Proliferation und Migration

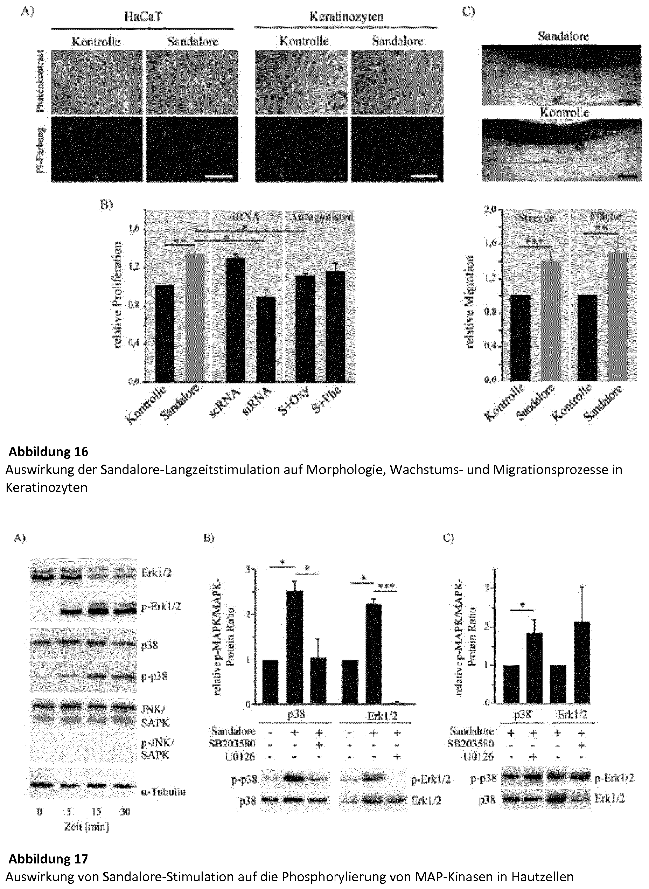

Um erste Hinweise über mögliche physiologische Effekte zu erhalten, welche durch die Aktivierung des OR2AT4 hervorgerufen werden, wurden HaCaT-Zellen und primäre Keratinozyten für fünf Tage mit Sandalore^{®} stimuliert. Hierbei wurden mögliche auftretende apoptotische oder nekrotische Auswirkungen durch Betrachtung der Morphologie der Zellen an einem Durchlichtmikroskop und einer anschließenden Propidiumiodid (PI)-Färbung, bei der nur die perforierte Membran nekrotischer oder apoptotischer Zellen passiert wird, aufgedeckt. Nach 5-tägiger Inkubation mit 500 µM Sandalore^{®} zeigten weder HaCaT-Zellen noch Keratinozyten eine morphologische Veränderung oder PI-Färbung. Ein letaler Effekt des Duftstoffs kann daher ausgeschlossen werden **(****Abbildung 16a****).**

Als nächstes wurde überprüft, ob Sandalore^{®} einen Einfluss auf das Wachstumsverhalten der Hautzellen hat. Dafür wurden HaCaT-Zellen erneut für fünf Tage mit Sandalore stimuliert und eine Zellzahlbestimmung mit Hilfe eines Proliferationsassays durchgeführt. Es zeigte sich, dass Sandalore^{®} die Proliferation der HaCaT-Zellen im Vergleich zur Kontrolle (0,1 % DMSO) um 33 % erhöhte **(****Abbildung 16b****).** Um zu verifizieren, dass diese Proliferationserhöhung über die Aktivierung des OR2AT4 induziert wird, wurden HaCaT-Zellen jeweils mit siRNA oder scRNA transfiziert und der Proliferationsassay erneut durchgeführt. HaCaT-Zellen, transfiziert mit scRNA, zeigten die gleiche Proliferationserhöhung wie untransfizierte Zellen. Die Transfektion der siRNA führte jedoch zur signifikanten Reduktion des durch Sandalore^{®} induzierten Effekts **(****Abbildung 16b****).**

Zudem wurde die Zellzahl nach Kostimulation (1:1) von Sandalore^{®} (500 µM) mit den Antagonisten Oxyphenylon (500 µM) oder Phenirat (500 µM) bestimmt. In Gegenwart des jeweiligen Antagonisten war die Proliferation reduziert. Für Oxyphenylon war der Effekt signifikant. Oxyphenylon und Phenirat alleine hatten keinen signifikanten Effekt auf die Proliferation der HaCaT-Zellen.

Insgesamt zeigten die Ergebnisse der siRNA- und Antagonisten-Experimente, dass die wachstumsfördernde Wirkung durch Sandalore^{®} über die Aktivierung des OR2AT4 vermittelt ist. Eine leicht erhöhte Proliferation von Keratinozyten kommt z.B. bei Reepithelialisierungsprozessen vor. Zusätzlich zur Proliferation ist bei diesem Prozess die Migration der Hautzellen leicht erhöht. Daher wurde mit Hilfe eines Agarose-MigrationsAssays überprüft, ob die Wachstumssteigerung der Zellen mit einem erhöhten chemotaktischen Verhalten einhergeht. Bei diesem Assay wurden HaCaT-Zellen auf einem Agarose-Medium- Gemisch ausgesät und die Ausbreitung der Zellen in Richtung 500 µM Sandalore^{®} bzw. 0,1 % DMSO für fünf Tage beobachtet. Nach Ausmessen der Strecke, welche die Zellen gewandert waren, bzw. der Fläche, welche die Zellen bewachsen hatten, ergab sich eine signifikante chemotaktische Migration der HaCaT-Zellen in Richtung Sandalore^{®} **(****Abbildung 16c****).**

Zusammenfassend zeigten die Ergebnisse, dass Sandalore^{®} über den OR2AT4 eine Signalkaskade induziert, die zu einer Erhöhung der Proliferation und Migration der HaCaTZellen führt.

### Auswirkung der Sandalore^{®}-Stimulation auf die Phosphorylierung von MAP-Kinasen

Mitogen-aktivierte Protein (MAP)-Kinasen spielen eine wichtige Rolle bei der Übertragung und der Umwandlung von extrazellulären Reizen in intrazelluläre Signale und regulieren dadurch zahlreiche Prozesse wie Zelldifferenzierung, -proliferation oder -stress. Die Signalwege umfassen mindestens drei nacheinander durch Phosphorylierung aktivierte Kinasen: eine vorgeschaltete MAP-Kinase-Kinase-Kinase (MAPKKK), eine nachgeschaltete MAP-Kinase-Kinase (MAPKK) und eine terminale MAP-Kinase (MAPK). Die MAPK-Signalwege werden nach ihren terminalen MAPK benannt, wobei die drei wichtigsten die Erk 1/2 (*extracellular signal-regulated kinase*)*-MAPK,* p38-MAPK und die JNK/SAPK (*c-Jun NH2-terminal kinase*/*stress-activated phospho-kinases)* sind.

Im Folgenden sollte untersucht werden, ob und wenn ja welcher MAPK-Signalweg durch Sandalore^{®}-Stimulation in Hautzellen aktiviert wird. Zum Nachweis wurden HaCaT-Zellen für 0-30 min mit 500 µM Sandalore^{®} stimuliert, die Proteine isoliert und in Western Blot-Analysen verwendet. Die Ergebnisse des Western Blots zeigten eine zunehmende Phosphorylierung der Erk1/2-MAPK sowie der p38-MAPK innerhalb von 5-30 min, jedoch keine Phosphorylierung der durch zellulären Stress induzierten JNK/SAPK **(****Abbildung 17a****).** Die Quantifizierung der Western Blot-Analysen von HaCaTZellen zeigte eine signifikante Zunahme der Phosphorylierung der Erk1/2-MAPK sowie der p38-MAPK bei 30-minütiger Sandalore^{®}-Stimulation. Bei zusätzlicher Inkubation der MAPKInhibitoren SB203580 für die p38-MAPK und U0126 für die Erk1/2- MAPK war die Phosphorylierung wieder signifikant reduziert **(****Abbildung 17b****).**

Um eine mögliche Wechselwirkung der beiden MAPK-Signalwege aufzudecken, wurde die Erk1/2-Phosporylierung in Anwesenheit des p38-MAPK-Inhibitors SB203580 und die p38- MAPK-Phosporylierung in Anwesenheit des Erk1/2-MAPK-Inhibitors U0126 untersucht. Die Phosphorylierung war für beide Kinasen in Anwesenheit des jeweiligen Inhibitors relativ zu den nur mit Sandalore^{®} stimulierten Zellen erhöht. Für die p38-MAPK war dieser Effekt signifikant **(****Abbildung 17c****).**

Zusammenfassend zeigten die Ergebnisse, dass die p38- und Erk1/2-MAPK durch Sandalore^{®} Stimulation phosphoryliert werden und eine gegenseitige Regulation der Signalwege besteht.

### Auswirkung von Sandalore-Stimulation auf humane Keratinozyten in einem in vitro "Wundheilungs"-Assay

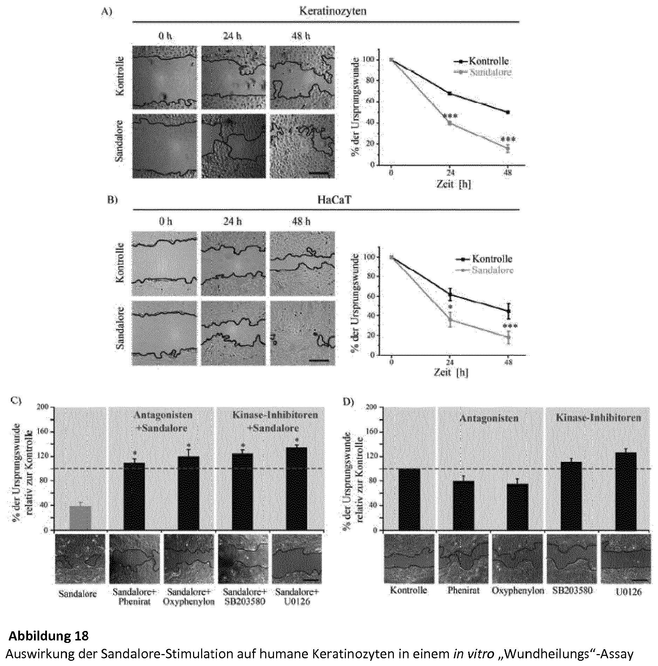

Die MAP-Kinasen p38 und Erk1/2 stehen oft im Zusammenhang mit erhöhter Proliferation und Migration in epidermaler Wundheilung. Daher sollte in einem weiteren physiologischen Experiment die Auswirkung von Sandalore auf die "Wundheilung" einer konfluenten Zellschicht aus HaCaT-Zellen oder primären Keratinozyten untersucht werden. Hierzu wurde ein Wund-Kratz-Assay verwendet, der eine etablierte *in vitro* Methode für erste Untersuchungen von Wundheilungsprozessen in Keratinozyten darstellt. In dem durchgeführten Wund-Kratz-Assay wurde ein Streifen in eine konfluente Zellschicht von HaCaT-Zellen bzw. primären Keratinozyten mit einer Pipettenspitze gekratzt und diese "Wunde" für zwei Tage beobachtet. Die Ergebnisse zeigten, dass sich dieser Kratzer bei HaCaT-Zellen um 26 % und bei Keratinozyten um 34 % schneller schließt, wenn die Zellen mit 500 µM Sandalore^{®} behandelt wurden **(Abbildungen 18a und 18b).**

Zur Überprüfung der Beteiligung des OR2AT4 Rezeptors an diesem Effekt wurde untersucht, ob die beiden Antagonisten Oxyphenylon und Phenirat die durch Sandalore^{®} erhöhte "Wundheilung" wieder reduzieren können. Hierzu wurde Sandalore^{®} mit jeweils einem der Antagonisten im gleichen Verhältnis gemischt und der Effekt auf die "Wundheilung" der HaCaT-Zellen erneut für 48 Stunden beobachtet. Es zeigte sich, dass durch die Koinkubation die "Wundheilung" wieder auf das normale Level reduziert wurde **(****Abbildung 18c****).** Die beiden Antagonisten alleine hatten keinen Einfluss auf die "Wundheilung" (Abbildung 18d) Die Beteiligung der MAP-Kinasen p38 und Erk1/2 wurde mittels spezifischer Inhibitoren der Kinasen überprüft. Für die p38-MAPK wurde der Erk1/2-Inhibitor SB203580 und für die Erk1/2-MAPK der p38-Inhibitor U0126 verwendet. In Gegenwart der jeweiligen Inhibitoren war die "Wundheilung" ebenfalls wieder auf das normale Level reduziert **(****Abbildung 18c****)** und bestätigt die Beteiligung der p38-MAPK und Erk1/2-MAPK an den Sandalore^{®}-induzierten Wundheilungsprozessen. Die beiden Inhibitoren alleine hatten keinen Einfluss auf die "Wundheilung" **(****Abbildung 18d****).**

### Untersuchung der Ausschüttung von Interleukinen sowie Phosphorylierung der Akt-Proteinkinase

Wie bereits vorstehend gezeigt, wirkt sich Sandalore^{®} positiv auf die "Wundheilung" der kultivierten Keratinozyten in einem *in vitro* Wund-Assay aus. Die Wundheilung ist ein komplexer Vorgang, der durch verschiedene physiologische Prozesse charakterisiert ist. Um weitere Hinweise darüber zu erhalten, wie die OR2AT4-aktivierte Signalkaskade zur erhöhten Wundheilung der Hautzellen beiträgt, sollte die Produktion und Ausschüttung von Interleukinen nach Sandalore^{®}-Stimulation mittels quantitativer *real-time-*PCR sowie eines ELISA-Assays in HaCaT-Zellen untersucht werden. Weiterhin sollte mittels Western Blot-Analysen überprüft werden, ob die Proteinkinase Akt, die in Keratinozyten an Differenzierungsprozessen beteiligt ist, durch Sandalore^{®}-Stimulation phosphoryliert wird.

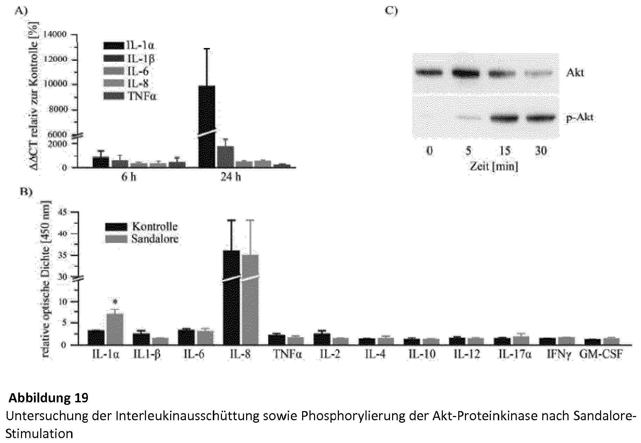

Zur Untersuchung der Interleukinausschüttung wurde zunächst eine quantitative *real-time-*PCR durchgeführt. Hierzu wurde RNA von HaCaT-Zellen verwendet, die zuvor für 6 bzw. 24 Stunden mit Sandalore^{®} stimuliert wurden. Der Nachweis erfolgte mit spezifischen Primern für verschiedene Interleukine (IL-1α, IL-1β, IL-6, IL-8) und dem Tumornekrosefaktor (TNF·). Es zeigte sich, dass nach 24 Stunden die Expression von IL-1α um ca 10.000 % und von Interleukin IL-1β um 1700 % relativ zur Kontrolle (0,1 % DMSO) erhöht war. Für die anderen Interleukine zeigte sich keine Erhöhung der Expressionsrate **(****Abbildung 19a****).** Zur Unterstützung der Ergebnisse wurde ein ELISA-Assay durchgeführt, bei dem ausgeschüttete Interleukine im Überstand der Zellen mittels einer immunologischen Reaktion nachgewiesen werden können. Mit dem verwendeten Assay konnte die Ausschüttung der Interleukine (IL-1α, IL-1β, IL-2, IL-4, IL-6, IL-8, IL-10, IL-12 und IL-17α) sowie des TNFa, des Gamma-Interferon IFNγ und des *Granulocyte macrophage colony-stimulating factor* (GMCSF) untersucht werden. Nach 24-stündiger Stimulation mit Sandalore^{®} war nur für IL-1α eine signifikant erhöhte Ausschüttung relativ zur Kontrolle (0,1 % DMSO) sichtbar. HaCaT-Zellen zeigten eine sehr hohe Ausschüttung von IL-8, jedoch war diese nicht signifikant höher als die Kontrolle (0,1 % DMSO) **(****Abbildung 19b****).**

Zusätzlich zu den bereits nachgewiesenen phosphorylierten Kinasen wurde die Phosphorylierung der Proteinkinase Akt untersucht. Hierzu wurden HaCaT-Zellen für 0, 5, 15 und 30 min mit Sandalore^{®} stimuliert und die Phosphorylierung mittels Western Blot-Analysen und speziellen Antikörpern, die gegen die unphosporylierte bzw. phosphorylierte Form des Akt-Proteins gerichtet sind, untersucht. Es zeigte sich, dass die Akt-Kinase innerhalb von 5 min durch Sandalore^{®}-Stimulation phosphoryliert wird und der Effekt bei längerer Stimulation (15 bzw. 30 min) weiter zunimmt **(****Abbildung 19c****).**

Zusammenfassend belegten die Ergebnisse, dass die Expression sowie Ausschüttung von IL-1α durch Sandalore^{®} erhöht war und die Proteinkinase Akt, die an Differenzierungsprozessen von humanen Keratinozyten beteiligt ist, aktiviert wurde.

### ERLÄUTERUNG DER NACHFOLGENDEN ABBILDUNGEN

Nachfolgend wird die vorliegende Erfindung durch eine Zahl von Abbildungen näher illustriert, deren Inhalt nachfolgend erläutert wird.

### Abbildung 1

**Heterologe Expression des OR2AT4 in Hana3A-Zellen.** Die transient mit OR2AT4 transfizierte Hana3A-Zellen (obere Reihe) zeigten bei Verwendung eines α-Rho-AKs (rot) und α -OR2AT4-AKs (grün) eine deutliche Überlagerung in der immuncytochemischen Färbung. Bei Kontrollfärbungen von transient mit OR1A2 transfizierten Hana3A-Zellen (untere Reihe) war hingegen nur eine Rhodopsin-Färbung erkennbar. Zur Bestimmung der Lage und Anzahl der Zellen wurde parallel eine Kernfärbung (DAPI, blau) durchgeführt. Die Fluoreszenzbilder wurden mit einem konfokalen Mikroskop aufgenommen. Maßstab: 20 µm.

### Abbildung 2

**Membranexpression des OR2AT4 in Hana3A-Zellen.** Abbildung 2a zeigt die Lebendzellfärbung von Hana3A-Zellen mit einem α-Rho-AK. *Links:* Transient mit OR2AT4 transfizierte Hana3A-Zellen zeigten eine Membranfärbung. *Rechts:* Untransfizierte Hana3A-Zellen zeigten keine Rhodopsin- Färbung. Die Fluoreszenzbilder wurden mit einem konfokalen Mikroskop aufgenommen. Maßstab: 20 µm. In Abbildung 2b ist die Quantifizierung der Lebendzellfärbung wiedergegeben. Für die Bestimmung der Transfektionsrate wurde die Gesamtzellzahl (Gesamt) sowie die Anzahl der transfizierten (transf.) Zellen an einem Fluoreszenzmikroskop ausgezählt. Dargestellt sind die mittleren Zellzahlen von drei unabhängigen Transfektionen inklusive Standardfehler.

### Abbildung 3

**Bestimmung des rezeptiven Feldes des rekombinant exprimierten OR2AT4.** Abbildung 3a zeigt die repräsentativen *Calcium Imaging-*Spuren von transient OR2AT4-exprimierenden Hana3A-Zellen, die zweimal (je 20 s, waagerechte Balken) mit Sandalore (1 mM) stimuliert wurden. Als Kontrolle wurden untransfizierte Hana3A-Zellen mit Sandalore stimuliert. 100 µM ATP diente am Ende jeder Messung als positiver Kontrollstimulus. In Abbildung 3b findet sich die Auswertung der *Calcium Imaging*-Messungen, in denen transient OR2AT4-exprimierende Hana3AZellen mit verschiedenen Sandelholzdüften (1 mM) stimuliert wurden. Die mittlere Antwortrate inklusive Standardfehler der Zellen ist relativ zur Ringer-Kontrolle dargestellt. Anzahl der Messungen: Ringer (23), Sandalore^{®} (22), Brahmanol^{®} (14), Javanol (15), Sandranol (18), Polysantol (14), Isobornylcyclohexanol (14), Sandelholzöl (14). Isobornyl.: Isobornylcyclohexanol; S. öl: Sandelholzöl. Kruskal-Wallis-Test mit multiplem Vergleich. ^{∗}: p<0,05. In Abbildung 3c ist die Auswertung des dualen Luciferase-Assays wiedergegeben. Die Signifikanz wurde zwischen Hana3A Zellen transfiziert mit OR2AT4-pcDNA3 bzw. pcDNA3 ohne Insert bestimmt. Die Punkte zeigen die mittlere, normalisierte Luciferase-Aktivität inklusive Standardfehler. Mann-Whitney-U-Test; n=3 Experimente. ^{∗}: p<0,05; ^{∗∗}: p<0,01; ^{∗∗∗}: p<0,001.

### Abbildung 4

**Antagonisten des rekombinant exprimierten OR2AT4.** Die Abbildung zeigt repräsentative *Calcium Imaging-*Spuren von transient OR2AT4-transfizierten Hana3A-Zellen, die zweimal (je 20 s, waagerechte Balken) mit Sandalore^{®} (blaue Spuren), Sandalore^{®} + Dimetol (hellblaue Spuren), Sandalore + Phenirat (graue Spuren) bzw. Sandalore + Oxyphenylon (schwarze Spuren) stimuliert wurden. 100 µM ATP diente am Ende jeder Messung als positiver Kontrollstimulus. In Abbildung 4b ist die Auswertung der *Calcium Imaging*-Messungen wiedergegeben, in denen transient OR2AT4-exprimierende Hana3AZellen mit verschiedenen Duftstoffen (1 mM) stimuliert wurden. Die mittlere Antwortrate inklusive Standardfehler der Zellen ist relativ zur Ringer-Kontrolle dargestellt. Anzahl der Messungen: Ringer (23), Sandalore^{®} (22), Sandalore^{®} + Dimetol (11), Dimetol (11), Phenirat (14), Sandalore^{®} + Phenirat (22), Oxyphenylon (12) und Sandalore^{®} + Oxyphenylon (20). S: Sandalore^{®}. Kruskal-Wallis-Test mit multiplem Vergleich. ^{∗}: p<0,05.

### Abbildung 5

**Rezeptives Feld des OR2AT4 sowie Strukturformeln der Duftstoffe.** Das rezeptive Feld umfasst die Agonisten (innerer Kreis), die inaktiven Substanzen (grauer Kreis) sowie die Antagonisten (oberes Rechteck). Für das getestete Sandelholzöl wurden exemplarisch die beiden Hauptkomponenten α-Santalol und β-Santalol abgebildet.

### Abbildung 6

**Expression des OR2AT4 in verschiedenen humanen Hautzelltypen und Geweben.** Zum Nachweis des OR2AT4 wurde eine PCR-Analyse mit cDNA verschiedener humaner RNA-Proben ("+") durchgeführt. Verunreinigung mit genomischer DNA konnten aufgrund einer - RT-Probe ("-") ausgeschlossen werden. Fragmentgrößen: OR2AT4 (400 bp), β-Aktin (250 bp). M: Marker, P: Zellkulturpassage.

### Abbildung 7

**Expression des OR2AT4 in HaCaT-Zellen und humanen primären Keratinozyten auf Proteinebene.** In Abbildung 7a sieht man HaCaT-Zellen sowie primäre Keratinozyten, die bei Verwendung eine sα-OR2AT4-AKs (rot) eine deutliche immuncytochemische Färbung zeigten. Abbildung 7b zeigt Kontrollfärbungen mit einem blockierenden Peptid (block. Peptid, Verhältnis des OR2AT4-AK zu blockierendem Peptid=1:2) war die Färbung stark reduziert. Zur Bestimmung der Lage und Anzahl der Zellen wurde parallel eine Kernfärbung (DAPI, blau) durchgeführt. Die Fluoreszenzbilder wurden mit einem konfokalen Mikroskop aufgenommen. Maßstab: 20 µm.

### Abbildung 8

**Expression des OR2AT4 in menschlichen Hautschnitten.** In **Abbildung 8a** zeigten die epidermalen Keratinozyten bei Verwendung des α-OR2AT4-AKs (grün) eine deutliche immuncytochemische Färbung. **Abbildung 8b** zeigt die Kontrollfärbungen, in denen der Erstantikörper durch Kaninchen- Serum ersetzt wurde; hier war die Färbung stark reduziert. *Oben:* 400-fache Vergrößerung; *unten:* Ausschnitt. E: Epidermis, D: Dermis, B: Basalschicht.

### Abbildung 9

**Effekt des OR2AT4-Liganden Sandalore^{®} auf die intrazelluläre Calciumkonzentration von kultivierten Keratinozyten.** Sowohl in A) primären Keratinozyten (n=175 Zellen) als auch in B) HaCaT-Zellen (n=186 Zellen) induzierte Sandalore (500 µM) bei repetitiver Stimulation (waagerechte Balken) Calciumsignale in *Calcium Imaging-*Experimenten. *Links:* Repräsentative *Calcium Imaging-*Spur der Sandalore-induzierten Calciumsignale. 100 µM ATP diente am Ende jeder Messung als positiver Kontrollstimulus. *Rechts:* Quantitative Auswertung der Calciumsignale bei 4-facher Applikation von Sandalore (500 µM). Dargestellt sind die Mittelwerte der einzelnen Applikationen inklusive Standardfehler. Mann-Whitney-U-Test. ^{∗}: p<0,05. C) und D) Dosisabhängige Aktivierung von HaCaT-Zellen in *Calcium Imaging*-Experimenten. Dargestellt sind die gemittelten Amplituden inklusive Standardfehler bei verschiedenen Konzentrationen bei der ersten (C) und vierten (D) Sandalore-Applikation. Anzahl der gemessenen Zellen: 0,0125 mM (39), 0,025 mM (30), 0,1 mM (46), 0,5 mM (34), 1 mM (63), 2 mM (41), 10 mM (36).

### Abbildung 10

**Einfluss von *gap junction*-Blockern auf die Sensitisierung der Sandalore^{®}**-**induzierten Calciumsignale.** A) Repräsentative *Calcium Imaging-*Spuren von HaCaT-Zellen (links) und primären Keratinozyten (rechts), die mit dem *gap junction-*Blocker 1-Octanol (500 µM) inkubiert und anschließend repetitiv mit Sandalore^{®} (500 µM, waagerechte Balken) stimuliert wurden (blaue Spur). Die schwarze Spur zeigt die repetitive Sandalore^{®}-Stimulation ohne Vorinkubation mit Blocker (Kontrolle). 100 µM ATP diente am Ende jeder Messung als positiver Kontrollstimulus. Quantitative Auswertung von HaCaT-Zellen (B) und primären Keratinozyten (C), die mit den *gap junction-*Blockern 1-Octanol (500 µM) bzw. Carbenoxolon (10 µM) inkubiert und anschließend mit Sandalore^{®} (500 µM) bzw. Lyral (1 mM) repetitiv in *Calcium Imaging*-Experimenten stimuliert wurden. Dargestellt sind die Mittelwerte der einzelnen Applikationen inklusive Standardfehler relativ zu KontrollMessungen (Sandalore^{®}-Applikation ohne Blocker). Mann-Whitney-U-Test. Anzahl der gemessenen Zellen: Sandalore + 1-Octanol (HaCaT (110), Keratinozyten (26)); Sandalore^{®} + Carbenoxolon: HaCaT (74); Lyral + Carbenoxolon: HaCaT (43). ^{∗∗∗}: p<0,001.

### Abbildung 11

**Pharmakologische Charakterisierung der Sandalore^{®}-induzierten Signalkaskade in humanen Keratinozyten.** A)-D) Dargestellt sind repräsentative *Calcium Imaging-*Spuren von primären Keratinozyten in Anwesenheit eines Blockers (blau). Die waagerechten Balken entsprechen den Applikationszeiten. 100 µM ATP diente am Ende jeder Messung als positiver Kontrollstimulus. A) Messung mit calciumfreier extrazellulärer Lösung (+10 mM EGTA). B) Messungen mit dem Adenylylcyclase-Blocker SQ-22536 (100 µM). C) Verwendung des Phospholipase-C-Blockers U-73122 (10 µM). 100 µM Histamin wurde als Positivkontrolle verwendet. D) Messungen mit dem CNG-Kanal-Blocker *L-cis* Diltiazem (150 µM). E) Übersicht der pharmakologischen Messungen an HaCaT-Zellen und primären Keratinozyten (Kera). Es sind die mittleren Amplituden relativ zu Kontrollmessungen (gestrichelte Linie) angegeben. MDL-12330A (40 µM). EGTA: calciumfreier Ringer (HaCaT n=46, Kera n=52), SQ: SQ-22536 (HaCaT n=60, Kera n=26), MDL: MDL-12330A (HaCaT n=69, Kera n=24), U-73122 (HaCaT n=41, Kera n=25), L-*cis*-D.: L*-cis* Diltiazem (HaCaT n=41, Kera n=25). Mann-Whitney-U-Test. ^{∗∗∗}: p<0,001. F) Bestimmung des cAMP-Gehalts von HaCaT-Zellen nach Stimulation mit Sandalore mit Hilfe des cAMP-GloTM Assays. Die Punkte zeigen den mittleren cAMP-Gehalt inklusive Standardfehler nach 10-minütiger Sandalore^{®}-Stimulation (1 µM- 10000 µM). Die Signifikanz wurde zwischen HaCaT-Zellen stimuliert mit Sandalore^{®} bzw. stimuliert mit einem äquivalenten Volumen an DMSO (0,2 %) bestimmt. Kruskal-Wallis Test mit multiplem Vergleich; n=3 Experimente. ^{∗}: p<0,05.

### Abbildung 12

**Expressionsanalyse von Signalkaskadenkomponenten und strukturverwandten Proteinen mittels NGS-Daten und RT-PCR.** A) Übersicht der NGS-Ergebnisse und der durchgeführten RT-PCR. Die Ergebnisse der NGS-Daten sind als FPKM-Werte angegeben. Ein erfolgreicher Nachweis der Transkripte in RT-PCR Experimenten ist mit (✔) gekennzeichnet; ein ausbleibender Nachweis mit (x). k.A.: keine Angaben. Komponenten der olfaktorischen Signalkaskade sind mit (^{∗}) gekennzeichnet. B) RT-PCR verschiedener Signalkaskadenkomponenten. Fragmentgrößen: AC3 (311 bp), ANO1 (288 bp), ANO6 (432 bp), ANO8 (427 bp), ANO9 (416 bp), ANO10 (373 bp), CNGA1 (2790 bp), CNGB1 (288 bp), GNAL (485 bp). M: Marker, K: Kontrolle, GNAL: GαolfR- Untereinheit, GNAS: G·SR-Untereinheit, ADCY: Adenylylcyclase, ANO: Anoctamin (Transmembranprotein16).

### Abbildung 13

**Expressionsanalysen der AC3 und des CNGA1 mittels immuncytochemischer Färbungen.** A) HaCaT-Zellen sowie primäre Keratinozyten zeigten bei Verwendung der spezifischen Antikörper α-Gaolf, α-CNGA1 und α-AC3 eine deutliche immuncytochemische Färbung. Als Kontrolle dienten Färbungen, bei denen nur der Zweitantikörper verwendet wurde. Zur Bestimmung der Lage und Anzahl der Zellen wurde parallel eine Kernfärbung (DAPI, blau) durchgeführt. Die Fluoreszenzbilder wurden mit einem konfokalen Mikroskop aufgenommen. Maßstab: 20 µm. B) Expressionsnachweis der AC3 in menschlichen Hautschnitten mittels immuncytochemischer Färbung. Epidermale Keratinozyten zeigten bei Verwendung des α-AC3-AKs (grün) eine deutliche immuncytochemische Färbung. Bei Kontrollfärbungen, in denen der Erstantikörper durch Kaninchen-Serum ersetzt wurde, war keine spezifische Färbung erkennbar. 400-fache Vergrößerung. E: Epidermis, D: Dermis, B: Basalschicht.

### Abbildung 14

**RNA-Interferenz-Versuche zur Verifizierung der Beteiligung des OR2AT4 an Sandalore-induzierten Calciumsignalen in HaCaT-Zellen.** A) Dualer Luciferase-Assay zur Bestimmung der siRNA-Aktivität in Hana3A-Zellen. Dargestellt ist das normalisierte Lumineszenzsignal (*Firefly*/*Renilla*) von Hana3A-Zellen kotransfiziert mit siRNA oder scRNA und dem Reportervektor pmirGLO-OR2AT4 relativ zu Zellen, die nur mit dem pmirGLO-OR2AT4 transfiziert wurden. Mann-Whitney-U-Test, n=3 Experimente. ^{∗∗∗}: p<0,001. B) *Links:* Immuncytochemische Färbungen von HaCaT-Zellen transfiziert mit siRNA- oder scRNAexprimierenden Plasmiden und dem α-OR2AT4-AK (rot). siRNA- bzw. scRNAexprimierende Zellen können anhand der GFP-Expression (grün) identifiziert werden. *Rechts:* Die quantitative Auswertung zeigte eine signifikante Reduktion der α-OR2AT4-Färbung der siRNA-exprimierenden Zellen (n=17 Zellen) relativ zu scRNA-exprimierenden Zellen (n=17 Zellen). Dargestellt sind die mittleren Fluoreszenzintensitäten inklusive Standardfehler der siRNA- relativ zu scRNA-exprimierenden Zellen. Mann-Whitney-U-Test. ^{∗∗}: p<0,01. C) Durchlicht- und Fluoreszenzbild von HaCaT-Zellen transfiziertmit einer Mischung aus den beiden siRNA. Maßstab: 50 µm. D) Fluoreszenzbilder einer *Calcium Imaging*-Messung. Die siRNA-exprimierende Zelle (Kreis) konnte durch Koexpression von GFP identifiziert werden und zeigte keine Erhöhung der intrazellulären Calciumkonzentration bei Stimulation mit Sandalore. E) Repräsentative *Calcium Imaging-*Spur von HaCaT-Zellen transfiziert mit siRNA (blaue Spur) oder scRNA (schwarze Spur), die repetitiv mit Sandalore^{®} (500 µM, waagerechte Balken) stimuliert wurden. 100 µM ATP diente am Ende der Messung als Kontrollstimulus. F) Quantitative Auswertung der Sandalore-induzierten Calciumsignale in siRNA- und scRNAexprimierenden Zellen. Die Amplituden der Calciumsignale wurden erst auf ATP normiert und dann die siRNA-exprimierenden Zellen relativ zu scRNA-exprimierenden HaCaT-Zellen ausgedrückt. Dargestellt sind die Mittelwerte der einzelnen Applikationen inklusive Standardfehler. Mann-Whitney- U-Test; n=18 Zellen (siRNA), n=17 Zellen (scRNA). ^{∗}: p<0,05; ^{∗∗}: p<0,01.

### Abbildung 15

**Wirkung der Antagonisten des OR2AT4 auf Sandalore^{®}-induzierte Calciumsignale in HaCaT-Zellen.** A) Repräsentative *Calcium Imaging-*Spuren von HaCaT-Zellen, die repetitiv mit Sandalore (1 mM) stimuliert wurden. Bei der dritten Applikation erfolgte eine Stimulation von Sandalore (dunkelblaue Spur) bzw. eine Kostimulation von Sandalore^{®} + Dimetol (hellblaue Spur), Sandalore^{®} + Oxyphenylon (schwarze Spur) oder Sandalore^{®} + Phenirat (graue Spur). 100 µM ATP diente am Ende jeder Messung als Kontrollstimulus. Die waagerechten Balken entsprechen den Applikationszeiten. Duftstoffe: 1 mM. B) Quantitative Auswertung der Calciumsignale bei alleiniger Sandalore^{®}-Stimulation bzw. bei Kostimulation mit Dimetol, Phenirat oder Oxyphenylon. S.: Sandalore^{®} Dargestellt sind die Mittelwerte der dritten Applikationen inklusive Standardfehler. Mann-Whitney-UTest. ^{∗∗∗}: p<0,001. Anzahl der gemessenen Zellen: Sandalore^{®} (121), Sandalore^{®} + Oxyphenylon (43), Sandalore^{®} + Phenirat (67), Sandalore + Dimetol (120). C) Untersuchung der dosisabhängigen Inhibition der Sandalore^{®}-induzierten Calciumsignale durch die Antagonisten Oxyphenylon und Phenirat. HaCaTZellen wurde bei der dritten Applikation mit verschiedenen Konzentrationen von Sandalore (500-1000 µM) und jeweils eines Antagonisten (100-500 µM) kostimuliert. Zur Vergleichbarkeit der Duftstoff-induzierten Calciumsignale wurden die Amplituden der dritten Applikation zur vorhergehenden Applikation normiert. Dargestellt sind die Mittelwerte inklusive Standardfehler der normierten Duftstoff-induzierten Calciumsignale relativ zu Kontrollzellen (Sandalore^{®}-Applikation ohne Antagonisten). Mann-Whitney-U-Test. ^{∗∗∗}: p<0,001. D) Berechnung der IC50-Werte für beide Antagonisten aus den unter C) dargestellten Daten für 500 µM.

### Abbildung 16

**Auswirkung der Sandalore-Langzeitstimulation auf Morphologie, Wachstumsund Migrationsprozesse in Keratinozyten.** A) Phasenkontrastbild und PI-Färbungen von HaCaTZellen und primären Keratinozyten nach Stimulation mit Sandalore^{®} (500 µM) bzw. 0,1 % DMSO (Kontrolle) für fünf Tage. Bei einer positiven PI-Färbung erscheinen Zellkerne rot gefärbt. Maßstab: 100 µm. B) Auswertung des Proliferationsassays durchgeführt mit dem CyQuant Cell Proliferation Kit an HaCaT-Zellen nach 5-tägiger Stimulation mit 500 µM Sandalore oder 0,1 % DMSO (Kontrolle). Für die RNA-Interferenz-Experimente wurden die Zellen am Tag vor der Sandalore-Stimulation mit siRNA bzw. scRNA transfiziert. Dargestellt ist die mittlere Zellzahl inklusive Standardfehler relativ zu unbehandelten Zellen. Mann-Whitney-U-Test; n=3 Experimente. ^{∗}: p<0,05; ^{∗∗}: p<0,01. S: Sandalore. C) Agarose-Assay zur Bestimmung der Migration von HaCaT-Zellen. *Oben:* Durchlichtbilder von HaCaT-Zellen, die in Richtung Sandalore (500 µM) bzw. 0,1 % DMSO gewandert sind. Die Wachstumsgrenze der Zellen wurde schwarz umrandet. Maßstab: 250 µm. *Unten:* Auswertung des Migrationsassays. Die längste gewanderte Strecke der Zellen sowie die bewachsene Fläche wurden ausgemessen und in Relation zur Kontrolle (0,1 % DMSO) gesetzt. Gepaarter zweiseitiger T-Test. Mittelwerte inklusive Standardfehler; n=4 Experimente. ^{∗∗}: p<0,01; ^{∗∗∗}: p<0,001.

### Abbildung 17

**Auswirkung von Sandalore^{®}-Stimulation auf die Phosphorylierung von MAPKinasen in Hautzellen.** A) Western Blot zur Überprüfung der Phosphorylierung der MAP-Kinasen in HaCaT-Zellen, die 0, 5, 15 und 30 min mit Sandalore (500 µM) stimuliert wurden. Zur Überprüfung der gleichmäßigen Proteinmenge wurde ▪-Tubulin als Kontrolle verwendet. Erk1/2: 42/44 kDa; p38: 43 kDa; JNK/SAPK: 46/54 kDA; ▪-Tubulin: 52 kDa. B) Quantitative Auswertung der p38- und Erk1/2- Phosphorylierung von HaCaT-Zellen, die 30 min mit Sandalore (500 µM) bzw. zusätzlich mit dem p38 Inhibitor SB203580 (10 µM) oder Erk1/2 Inhibitor U0126 (10 µM) stimuliert wurden. Zur Auswertung wurde die Ratio der phosphorylierten (p) und unphosporylierten Proteine gebildet und in Relation zu unbehandelten Zellen gesetzt. Mittelwert inklusive Standardfehler. Mann-Whitney-U-Test. ^{∗}: p<0,05; ^{∗∗∗}: p<0,001. In C) wurden die Inhibitoren der jeweils anderen MAP-Kinase verwendet und die Phosphorylierung in Relation zur den nur mit Sandalore^{®} (500 µM) stimulierten Zellen gesetzt. Mittelwert inklusive Standardfehler. Mann-Whitney-U-Test. ^{∗}: p<0,05. Die Western Blot-Analysen wurden mit drei unterschiedlichen Proteinisolierungen durchgeführt.

### Abbildung 18

**Auswirkung der Sandalore^{®}-Stimulation auf humane Keratinozyten in einem *in vitro* "Wundheilungs"-Assay.** Untersuchung der "Wundheilungsrate" von A) primären Keratinozyten und B) HaCaT-Zellen mittels eines Wund-Kratz-Assays in Anwesenheit von Sandalore (500 µM) oder 0,1 % DMSO als Kontrolle. *Links:* Durchlichtbilder der beobachteten "Wunde" zu verschiedenen Zeitpunkten. Die Wachstumsgrenze der Zellen wurde schwarz umrandet. Maßstab: 200 µm. *Rechts:* Quantitative Auswertung des Wund-Kratz-Assays. Die Flächen der Wunden wurden ausgemessen und in Relation zur anfänglichen Wunde (0 h) gesetzt. Mittelwert inklusive Standardfehler. Mann-Whitney- U-Test; n=4 Experimente. ^{∗}: p<0,05; ^{∗∗∗}: p<0,001. C) Wund-Kratz-Assay in Anwesenheit von Sandalore^{®} (500 µM) und jeweils einem der Antagonisten Oxyphenylon (500 µM) oder Phenirat (500 µM) bzw. des p38-MAPK-Inhibitors SB203580 (10 µM) oder des Erk1/2-MAPK-Inhibitors U0126 (10 µM). Die ausgemessenen Wundflächen der behandelten Zellen (nach 48-stündiger Stimulation) wurden in Relation zur Kontrolle (0,1 % DMSO) gesetzt. Mittelwert inklusive Standardfehler. Kruskal-Walis-Test mit multiplem Vergleich; n=3 Experimente. ^{∗}: p<0,05. D) Kontrollen zu dem unter C) durchgeführten Wund-Kratz-Assay mit Oxyphenylon (500 µM) oder Phenirat (500 µM) bzw. SB203580 (10 µM) oder U0126 (10 µM). Die ausgemessenen Wundflächen der behandelten Zellen wurden in Relation zur Kontrolle (0,1 % DMSO) gesetzt. Mittelwert inklusive.

### Abbildung 19

**Untersuchung der Interleukinausschüttung sowie Phosphorylierung der Akt-Proteinkinase nach Sandalore^{®}-Stimulation.** A) Untersuchung der Regulation von Interleukinen in HaCaT-Zellen mittels qRT-PCR nach Stimulation mit Sandalore^{®} (500 µM) für 6 h und 24 h. Als Kontrolle wurde 0,1 % DMSO verwendet. GAPDH wurde als Referenzgen verwendet und die relative Expression zwischen unbehandelten und stimulierten Zellen ermittelt. Mittelwert inklusive Standardfehler. n=3 Experimente. B) ELISA-Assay zur Detektion von Interleukinen im Überstand von HaCaT-Zellen, die 24 h Stunden mit Sandalore (500 µM) stimuliert wurden. Als Kontrolle wurde 0,1 % DMSO verwendet. Die gemessene optische Dichte bei 450 nm der behandelten Zellen wurde relativ zum Hintergrund ausgedrückt. Es wurden Überstände von drei unabhängigen Stimulationen verwendet. Mittelwert inklusive Standardfehler. Mann-Whitney-U-Test. ^{∗}: p<0,05. C) Western Blot zur Überprüfung der Phosphorylierung der Akt-Proteinkinase von HaCaT-Zellen, die 0, 5, 15 und 30 min mit Sandalore (500 µM) stimuliert wurden. Akt (60 kDA); p-Akt: phosphoryliertes Akt-Protein (60 kDA).

## Patentansprüche

1. Verfahren zur Identifizierung von Wirkstoffen, die die Wundheilung begünstigen, umfassend die folgenden Schritte:
(a) Bereitstellen einer Keratinozytenkultur, die den olfaktorischen Faktor OR2AT4 enthält,
(b) Hinzufügen des zu testenden Wirkstoffs zu der Kultur und
(c) Bestimmung der Veränderung der intrazellulären Calciumkonzentration.

2. Verfahren nach Anspruch 1, **dadurch gekennzeichnet, dass** man als Keratinozytenkultur eine Kultur von H-CaT-Zellen einsetzt.

3. Verfahren nach Anspruch 1, **dadurch gekennzeichnet, dass** man den Wirkstoff der Keratinozytenkultur mehrfach zufügt und die Veränderung der intrazellulären Calciumkonzentration über den Anstieg der Amplitude der Calciumkonzentration im Vergleich zur jeweils vorherigen Verabreichung bestimmt.

## Claims

1. A method for identifying agents that promote wound healing, comprising the following steps:
(a) providing a keratinocyte culture containing the olfactory factor OR2AT4,
(b) adding the agent to be tested to the culture; and
(c) determining the change in intracellular calcium concentration.

2. The method according to claim 1, **characterized in that** a culture of H-CaT cells is used as keratinocyte culture.

3. The method according to claim 1, **characterized in that** the active substance is added to the keratinocyte culture several times and the change in the intracellular calcium concentration is determined via the increase in the amplitude of the calcium concentration in comparison with the respective previous administration.

## Revendications

1. Procédé d'identification de substances actives favorisant la cicatrisation des plaies, comprenant les étapes suivantes :
(a) fournir une culture de kératinocytes contenant le facteur olfactif OR2AT4,
(b) ajout de l'agent à tester à la culture, et
(c) déterminer la variation de la concentration de calcium intracellulaire.

2. Procédé selon la revendication 1, **caractérisé en ce que** l'on utilise comme culture de kératinocytes une culture de cellules H-CaT.

3. Procédé selon la revendication 1, **caractérisé en ce que** l'on ajoute plusieurs fois la substance active à la culture de kératinocytes et que l'on détermine la modification de la concentration intracellulaire de calcium par l'augmentation de l'amplitude de la concentration de calcium par rapport à l'administration précédente respective.
